# EUROPEAN PATENT SPECIFICATION

(11) **EP 1 155 705 B1**
(45) Date of publication and mention of the grant of the patent: **02.04.2008**
(21) Application number: 01112106.8
(22) Date of filing: 17.05.2001
(51) Int. Cl.: A61L 27/12, A61L 27/56, A61L 27/38

(54) **Biomaterial**
Biomaterial
Biomatériau

(30) Priority: 19.05.2000 JP 2000148561; 27.09.2000 JP 2000294841; 27.09.2000 JP 2000294842; 27.09.2000 JP 2000294843
(43) Date of publication of application: 21.11.2001
(73) Proprietor: OCHI, TAKAHIRO, PH. D., KOBE-SHI, HYOGO (JP); MMT CO., LTD, OSAKA-SHI, OSAKA (JP); Covalent Materials Corporation, Shinagawa-ku Tokyo (JP)
(72) Inventor: Ochi, Takahiro, Ph.D., Kobe-shi, Hyogo (JP)
(74) Representative: Steil, Christian

(56) References cited:
- WO-A-98/15505
- GB-A- 2 078 696
- US-A- 4 371 484
- US-A- 4 654 314
- US-A- 5 318 779
- DATABASE WPI Section Ch, Week 199351 Derwent Publications Ltd., London, GB; Class B04, AN 1993-408357 XP002251764 & JP 05 305134 A (JAPAN STEEL WORKS LTD), 19 November 1993 (1993-11-19)
- DATABASE WPI Section Ch, Week 198912 Derwent Publications Ltd., London, GB; Class D21, AN 1989-088464 XP002251765 & JP 01 037475 A (ASAHI OPTICAL CO LTD), 8 February 1989 (1989-02-08)
- DATABASE WPI Section Ch, Week 199538 Derwent Publications Ltd., London, GB; Class L02, AN 1995-290172 XP002251766 & JP 07 187852 A (TOSHIBA CERAMICS CO), 25 July 1995 (1995-07-25)
- DATABASE WPI Section Ch, Week 199830 Derwent Publications Ltd., London, GB; Class J01, AN 1998-343138 XP002251767 & JP 10 130076 A (TOSHIBA CERAMICS CO), 19 May 1998 (1998-05-19)
- DATABASE WPI Section Ch, Week 199502 Derwent Publications Ltd., London, GB; Class D22, AN 1995-010903 XP002251768 & JP 06 296680 A (NGK SPARK PLUG CO LTD), 25 October 1994 (1994-10-25)
- DATABASE WPI Section Ch, Week 199514 Derwent Publications Ltd., London, GB; Class D22, AN 1995-100985 XP002251769 & JP 07 023994 A (MITSUBISHI MATERIALS CORP), 27 January 1995 (1995-01-27)
- LE HUEC J C ET AL: "Influence of porosity on the mechanical resistance of hydroxyapatite ceramics under compressive stress" BIOMATERIALS, ELSEVIER SCIENCE PUBLISHERS BV., BARKING, GB, vol. 16, no. 2, 1995, pages 113-118, XP004033091 ISSN: 0142-9612
- GAUTHIER O ET AL: "Macroporous biphasic calcium phosphate ceramics: influence of macropore diameter and macroporosity percentage on bone ingrowth" BIOMATERIALS, ELSEVIER SCIENCE PUBLISHERS BV., BARKING, GB, vol. 19, no. 1-3, 2 January 1998 (1998-01-02), pages 133-139, XP004118925 ISSN: 0142-9612

## Description

### FIELD OF THE INVENTION

The present invention relates to a biomember subserviently used in forming a bone in a living body, and a method for preparation thereof. For example, when a bone is defected by injury and disease, etc., the present invention relates to a biomember for regenerating its own bone by compensating the portion. In addition, the biomember can be used as sustained-release preparations (drug delivery system), and further, applied as carrier for cell culture in vitro. For example, when a bone for graft is prepared in vitro, it can be also used.

### DESCRIPTION OF THE RELATED ART

Regarding a bone defected by injury and disease, an artificial bone, a joint, etc. using metal or ceramics have been studied conventionally. And, as the ceramics, alumina, zirconia, etc. have been put to practical use in view of strength and no bio-harmfulness. Also, as the metal having excellent processability, titanium, etc. have been put to practical use in view of no bio-harmfulness.

However, since as to alumina, zirconia, titanium, etc. a harmless member is simply substituted for the bone as much as possible, they are dead-tissues (inert-tissues) incompatible with the living body even if a long time passes. Such tissues are not changed as a patient of growth phase or the aged patient grows older, that is, even if it is used for the patient of growth phase, it does not grow, and even if it is used for the aged patient, the patient may feel pain because it is not modified suitably for other bones. Further, if it is used for several years, release of ion etc. might occur. Though it is harmless, there may be anxious factors.

As other members, a calcium phosphates ceramics sintered body close to composition of an original bone, such as tricalcium phosphate, tetracalcium phosphate, hydroxyapatite, etc., has been put to practical use. The calcium phosphates ceramics is known to have no bio-harmfulness and to be easily compatible in vivo to slowly bond to its own tissue, and an osteoclast erodes the calcium phosphates ceramics, and then, its own bone may be formed in the eroded portion.

That is, once it is inserted by one operation, thereafter, it has excellent characteristics capable of being perfectly substituted for its own bone (or changed into its own bone). However, the calcium phosphates ceramics is weak in strength unless it is made as a compact body.

However, since it takes a long time for the compact body to be compatible with the living body or it remains in itself in vivo, it is impossible to make the most of original characteristics. In order to substitute the calcium phosphates ceramics for its own bone, it is preferable to use a porous body, but the porous body may be weak in strength and difficult for use.

Porous sintered bodies which can be used as artificial bones are known from US 4,654,314, GB 2 078 696, US 5,318,779, and US 4,371,484.

### SUMMARY OF THE INVENTION

An object of the present invention provides a biomember that has desired strength, and can remarkably enlarge a contact area with a living body or body fluids of blood etc. A method for preparation hereof is also provided.

With this biomember that cell infiltration into an inner surface is very fast, and a method for preparation thereof.

To the biomember a cell is easy to be attached. For that biomember an outstanding recuperative powers can be expected after operation.

A biomember according to the present invention is a porous body of a calcium phosphates sintered body having a number of substantially globular pores, and simultaneously, the porosity is not less than 65% and not more than 85%, and simultaneously, a mean pore diameter is not less than 100 µ m and not more than 600 µ m. A pore having a size larger than the mean pore diameter has at least four communicating pores having a diameter of not less than 5 µ m, on the average, and simultaneously, a pore having at least the four communicating pores has at least one communicating pore having a diameter of not less than 50 µ m, on the average. A total opening area of communicating pores which are possessed by a pore having a size larger than the mean pore diameter occupies the proportion of not more than 40% of a pore surface area, on the average. In a dry state, it is possible to wet the whole porous body by dropping water or blood.

Preferably, a pore having a size larger than a mean pore diameter has at least six communicating pores having a diameter of not less than 10 µm, on the average, and simultaneously, a pore having at least the six communicating pores has at least two communicating pores having a diameter of not less than 50 µm, on the average.

Also, a sum of flat areas of pores having a size larger than a mean pore diameter, which are shown in any plain cross sections is not less than 25% and not more than 60% of flat areas of all of the cross sections. Further, a sum of flat areas of pores having a size larger than a mean pore diameter, which are shown in any plain cross sections is not less than 35% and not more than 55% of flat areas of all of the cross sections.

Or, once a sintered body which is processed, washed and dried is only brought into contact with water (pure water) without performing pretreatment, the water infiltrates into a core part by a capillary phenomenon.

Also, once a sintered body which is processed, washed and dried is only brought into contact with blood (whole blood) without performing pretreatment, the blood infiltrates into a core part by a capillary phenomenon.

A thickness of a circumferential portion of a communicating pore formed by causing pores having a size larger than a mean pore diameter to overlap with each other is set to be about the thickness of one particle of calcium phosphates. In addition, the pore was formed from foaming by stirring of a slurry. Preferably, the calcium phosphates sintered body is hydroxyapatite.

Further, an osteogenic cell is introduced into a pore. Also, an automyelocyte is introduced into a pore. Also, a homogeneous myelocyte is introduced into a pore. And, a fetal myelocyte is introduced into a pore. Also, an undifferentiated stem cell is introduced into a pore.

Or, an osteogenic cell to which a gene of an active factor was introduced is introduced into a pore. Also, an automyelocyte to which a gene of an active factor was introduced is introduced into a pore. Also, a homogeneous myelocyte to which a gene of an active factor was introduced is introduced into a pore. Also, a fetal myelocyte to which a gene of an active factor was introduced is introduced into a pore. And, an undifferentiated stem cell to which a gene of an active factor was introduced is introduced into a pore.

Or, an active material is attached to an inner surface of pore. Also, the active material is referred to as a cell adhesive promoting material. And, the active material is referred to as a cell proliferation promoting material. Also, the active material is referred to as an osteogenesis promoting material. Also, the active material is referred to as a bone absorption inhibiting material. And, the active material is referred to as a vascularization promoting material. Or, the active material is a combination comprising at least two of the cell adhesive promoting material, cell proliferation promoting material, osteogenesis promoting material, bone adsorption inhibiting material and vascularization promoting material.

Further, an osteogenic cell is introduced into a pore that an active material is attached to the surface. Also, an automyelocyte is introduced into the pore. Or, a homogeneous myelocyte is introduced into the pore. And, a fetal myelocyte is introduced into the pore. Also, an undifferentiated stem cell is introduced into the pore.

Or, an osteogenic cell to which a gene of an active factor was introduced is introduced into a pore that an active material was attached to the surface. Also, an automyelocyte to which a gene of an active factor was introduced is introduced into a pore. Also, a homogeneous myelocyte to which a gene of an active factor was introduced is introduced into a pore. And, a fetal myelocyte to which a gene of an active factor was introduced is introduced into a pore. Also, an undifferentiated stem cell to which a gene of an active factor was introduced is introduced into a pore.

Also, drugs are stored in a pore, and the whole thereof is made into sustained-release preparations.

In the other mode of the present invention, it is preferable that a mean particle diameter of a calcium phosphate particle of slurry raw materials is of the submicron order (i.e., not less than 0.1 µ m and not more than 1 µ m), and it is preferable that the maximum particle diameter is also of the submicron order.

A porous body has a particle diameter of about 0.1 µ m in a dry state, and after sintering, it is preferable that the particle diameter grows to be about 2 - 3 µm.

The sintered porous body is in the state that grain growth is completed and adjacent particles are compactly contacted each other. The sintered porous body is a perfect sintered body comprising of substantially 100% of apatite (ie, hydroxyapatite).

In the present invention, the whole of raw particles is a submicron (about 0.1 µm) in diameter. The raw particles stabilize a pore shape by cross-polymerizable resin being a kind of polymer. A temperature in sintering is about 1,100°C. The submicron particles attain grain growth to be particles of not more than 5 µ m. A porous body after sintering comprises 100% of apatite. After sintering, unevenness of the particles on the surface of a pore wall is less, the surface is smooth, and adjacent particles are compactly contacted each other. A pore wall has a dense microstructure.

The porous body of the present invention is such that a skeleton becomes a compact apatite structure by grain growth of raw particles. The surface of a pore wall is smooth. The body is strong.

Also, for example, in order to infiltrate blood etc. into a pore, processing crumbs (powder) etc. should not be present on the surface. It becomes difficult for blood etc. to infiltrate. It is believed that the processing crumbs are surely produced during the processing required after sintering, however, if a communicating pore is small or the surface is sintered prematurely (insufficiently), it becomes difficult to entirely remove them up to a core part by washing, and thus, infiltration of blood etc. is interrupted.

The porous body of the present invention is such that because communicating pore communicate with each other and the inner surfaces are smooth, the processing crumbs are easily removed by washing.

Further, it is preferable that edges of a communicating pore are thin and sharp up to a size of one particle sintered.

According to the other mode of the present invention, by combining a biomember of an artificial joint etc. with the above-mentioned calcium phosphates sintered body, a biomember which can be unprecedentedly promptly integrated with a bone can be obtained.

Such a biomember is very useful as an artificial bone for a portion to which heavy load is applied or an artificial joint.

Because the biomember is comprised of a calcium phosphates sintered body, it can promote regeneration of a bone. If a pore is formed into a substantially globular shape as a whole, it has no directional property, and is easy to retain its strength. In addition, it can enlarge a surface area to which cells are attached.

Generally, a human cell has a size close to 10 µ m, an erythrocyte of an adult is also 8 - 9 µm in a diameter. If a communicating pore is 25 µ m in a diameter, the effects are sufficiently exhibited, and if a communicating pore is not less than 40 µ m in a diameter, the penetrating amount of oxygen, nutrients, cells, etc. is preferably enhanced. That is, a pore having at least three communicating pores (open pores) having a diameter of not less than 5 µm has preferably at least one communicating pore having a diameter of not less than 40 µm.

By having such a large communicating pore and three-dimensionally communicating with many pores, circulation of body fluids of the whole biomember inner part becomes better, and also, a cell becomes easy to infiltrate into a core part of the biomember.

Preferably, a pore having a size larger than a mean pore diameter has at least four communicating pores having a diameter of not less than 5 µ m, on the average on the inner surface, and such a pore has preferably at least one communicating pore on the average having a diameter of not less than 50 µm. More preferably, a pore having a size larger than the mean pore diameter has at least six communicating pores having a diameter of not less than 10 µm, on the average, and such a pore has at least two communicating pores having a diameter of not less than 50 µm, and therefore, circulation of body fluids into a pore becomes active. In addition, it is particularly preferable that a pore having at least two communicating pores having a diameter of not less than 50 µm has communicating pores having a diameter of not less than 80 µm.

It is preferable that a pore having a size larger than the mean pore diameter in a porous member is opened as the communicating pore in the ratio of not more than 50% of one round pore surface area, on the average. It is because if the pore inner surface area to be lost as a communicating pore becomes larger than 50%, a surface area to which a cell is attached becomes too small and it also affects the strength. Preferably, it is not more than 40%.

In explaining a pore having a size larger than the mean pore diameter, the pore having a size larger than approximately the mean pore diameter substantially affects extremely the effects etc.

The mean pore diameter of the present invention can be measured for example by a resin embedding. And, the 50% volume pore diameter i.e., a diameter of a pore when the value becomes exactly 50% of total pores by integrating volumes in a large pore (or a small pore) is referred to as the mean pore diameter.

Also, in any plain cross section, when observing a pore state in a plain aspect, it is preferable that a pore having a size larger than the mean pore diameter is 25 - 60% of a flat area. When it is less than 25%, a pore part becomes small, and thus, cell infiltration becomes difficult, and when it is more than 60%, it is liable to be weak in strength. More preferably, it is 35 - 55%. Even more preferably, it is 40 - 50%.

The biomember of the present invention is such that as a circumference part (i.e., the above-mentioned edge) of a communicating pore formed by causing pores to overlap with each other is formed sharply to the extent of the thickness of one calcium phosphate particle, it enlarges the surface area. Such characteristics of the pore can be obtained from foaming by stirring slurry raw material, and then, drying and sintering. At this time, it is preferable that the mean particle diameter of calcium phosphate particles of the slurry raw material is of the submicron order (i.e., not less than 0.1 µm and up to 1 µm), and it is preferable that the maximum particle diameter is also of the submicron order.

In the biomember of the present invention, a pore is not formed by baking and taking-out using globular particles of urethane etc. Thereby, pressure forming is not needed, there is no directional property that pores become plain etc., and simultaneously, compared with that a contact point of urethane is opened, in the biomember of the present invention, a communicating pore becomes remarkably large, and simultaneously, a surface area can be enlarged. For instance, Japanese Patent Laid-Open No. hei 10-167853 describes a porous body in which a circumferential part of a communicating pore is formed sharply, and because it is made by baking and taking-out, a communicating pore is as small as not more than 10 µm in diameter, and it is difficult for a cell to penetrate.

As one satisfying the above conditions, in the present invention, at least a porous member should be able to wet the inner surface of a pore in detail by water or blood in order that body fluid or cells can infiltrate easily.

In the present invention, because a pore of a porous member uses the one in the foregoing specific state, and the structure is homogeneous throughout the inner part, it is possible to wet the whole in a dry state by dropping water and blood.

By processing a sintered body if necessary, and then, washing and drying, for example, if a part thereof is immersed into water (pure water), such a porous member can soak water by a capillary phenomenon without pretreatment, even in a dry state. Also, it has characteristics that if water drops, it can flow into an inner part to reach a bottom part. Regarding blood (whole blood), it is the same as water.

The "in a dry state" means no treatment such as coating of surfactants or previous wetting by priming, etc., and it is possible to use without pretreatment for a living body. In addition, this expression is not the meaning of limiting a practical using method.

The porous member is installed in a portion where integration with a bone is needed. An artificial coxal bone articulation is a stem part. The thickness is related to a pore diameter, and thus, it is preferable to be not less than 300 µm, and it is more preferable to be not less than 500 µm. However, because the porous member is weak in strength, after integrating with a bone, it is preferable that it disappears by adsorption into the bone, and for this reason, it is preferable to be not more than 3 mm.

A compact member supports a load substantially, and is good as a member used in the conventional artificial joint, and for example, ceramics such as alumina, zirconia, etc., and metals such as titanium, titanium alloy, etc. are used. The compact member has a porosity preferably not more than 15%, and it is preferable that it has a porosity of not more than 5% even if the ceramics etc. Preferably, there are not substantially pores. If there is no problem in strength, it is preferable to use a compact body of calcium phosphates sintered body.

Even if such a compact body itself is buried into a bone, it generates a gap in a living body, or deviates, and therefore, it is not readily compatible. However, since its surface is made of a calcium phosphate porous body of the specific pore shape of the present invention, compatibleness with a bone becomes faster remarkably. It is believed that regeneration of a bone begins in a porous body and is integrated.

There is no specific restriction to methods of forming a porous member on the surface of a compact member, however, on the grounds that a thermal expansion coefficient is different etc. in a calcium phosphates sintered body and metals or ceramics of the other material, an intermediate layer may be installed therebetween because bonding and adhesion may be difficult.

The intermediate layer is preferably selected from ceramics of glass for a living body, calcium phosphate and calcium titanate. In particular, it is preferable that the intermediate layer is hydroxyapatite formed by spray coating.

If a biomember is an artificial joint and a porous member is the outer surface of a stem part, the effects of the present invention can be particularly exhibited. In this case, the compact member of the stem may have unevenness on the surface. The compact substance part of the stem may have pits or slits.

The porous member of the present invention may be formed by adhesion of the laminated shape, or may be also formed by adhesion of a calcium phosphates sintered body of the granular shape. After a hydroxyapatite layer is formed by spray coating on the outer surface of a compact substance member, an outer surface layer of a porous substance is adhered by intercalating a slurry, or foamed slurry forms a surface layer, and then, they may be integrated by thermal treatment or sintering.

Further, in order to accelerate integration with a bone and a biomember, active materials may be attached to the pore inner surface of a porous member, and osteogenic cells, automyelocytes, homogeneous myelocytes, fetal myelocytes or undifferentiated stem cells may be introduced into a pore. Furthermore, osteogenic cells to which a gene of an active factor is introduced, automyelocytes to which a gene of an active factor is introduced, homogeneous myelocytes to which a gene of an active factor is introduced, fetal myelocytes to which a gene of an active factor is introduced or undifferentiated stem cells to which a gene of an active factor is introduced, may be introduced into a pore. Also, various cells or various cells to which a gene is introduced may be used together with the active materials.

The biomember according to the other mode of the present invention is the biomember for regenerating its own bone by compensating the portion when a bone is defected or excised by injury or disease.

For example, as a member having at least a° compact part and a porous part comprised of a calcium phosphates sintered body, the compact part has a porosity of not less than 0% and not more than 50%, and the porous part has a porosity of not less than 55% and not more than 85%, and simultaneously, a pore of the porous part is comprised by assembling substantially globular pores, a mean pore diameter is not less than 50 µ m and not more than 800 µ m, a pore having a size larger than the mean pore diameter has at least three communicating pores (open pores) having a diameter of not less than 5 µ m, on the average, and simultaneously, such a pore has at least one communicating pore having a diameter of not less than 25 µm, on the average, and simultaneously, pores having a size larger than the mean pore diameter are opened in the ratio of not more than 50% of the pore surface area, on the average, and also, the porous part can wet the whole by dropping water or blood in a dry state.

The compact part has a porosity of not less than 0% and not more than 20%, at least pores of the porous part are formed by stirring and bubbling of a slurry, and the calcium phosphates sintered body is preferably hydroxyapatite.

Preferably, active materials are attached to the pore inner surface of a porous part of such a biomember. Also, at least one of osteogenic cells, automyelocytes, homogeneous myelocytes, fetal myelocytes and undifferentiated stem cells is introduced into a pore of a porous part of the biomember. Similarly, at least one of osteogenic cells to which a gene of an active factor is introduced, automyelocytes to which a gene of an active factor is introduced, homogeneous myelocytes to which a gene of an active factor is introduced, fetal myelocytes to which a gene of an active factor is introduced and undifferentiated stem cells to which a gene of an active factor is introduced, is preferably introduced into a pore of a porous part of the biomember. Meantime, it is possible to use as sustained release preparations by storing drugs into a pore of a porous substance.

If the compact part has a porosity of not more than 20%, the strength is enhanced preferably and sufficiently. In case where the compact part is hydroxyapatite, when the porosity is not more than 50%, bending strength becomes about 30-50 MPa, and when the porosity is not more than 20%, the bending strength becomes about 80-150 MPa, and therefore, heavy load is applied so that it gets to have the strength enough to support the load applied from the bone of a patient. Also, it can endure a stroke, and its handling can be easily performed in the operation etc.

If the compact part is not more than 50% of the whole volume of a biomember, it can take preferably and sufficiently a porous part to be substituted for a bone. If the compact part is formed in a part of the outer surface, for example, when it is buried in a bone, it can be preferably tapped and inserted.

If the compact part is formed in an inner part, while a porous part of outer surface is substituted for a bone in vivo, the compact part can maintain the strength of a biomember. If the compact part is formed in a part of the outer surface and inner part, the foregoing both effects are preferably obtained.

If the whole shape is a circular cone and a bottom surface is a compact part, it becomes a configuration usually used in an actual operation, and simultaneously, strike-installation is preferably possible. And, if an ampulla of a porous substance is set in the vicinity of an apex of the circular cone, a part of the ampulla is cut when it is inserted into a bone, and preferably, the cuttings fill up the remaining space.

If the whole shape is cylindrical and only peripheral side is a compact part, for example, an intermediate part of a rhabdome bone such as a femur, humerus, etc. can be preferably formed only by the biomember of the present invention.

If the whole shape is rectangular and only one side of outer surfaces is a compact part, for example, it is preferably suitable to be buried into a part of a large bone. Further, the compact part may be installed vertically toward an inner part from a stroke part to transmit a stroke power to the inner part.

A compact part is formed at the whole one side to transmit an impact by a plastic hammer etc. to the biomember as a whole, and its thickness is preferably not less than about 3 mm. When a surface area of the compact part is made much larger, pits may be installed in the compact part so as not to interrupt penetration of blood or cells with respect to a porous part. And, the pits may be filled with porous substances.

In the biomember of the present invention, in a pore shown in any plain cross section of a part of the porous substances, a sum of flat areas of pores having a pore diameter larger than the mean pore diameter is preferably 25-60% of flat areas of the total cross sections. In up to 25%, since pore parts become smaller, cell infiltration becomes difficult, and in more than 60%, it is liable to be weak in strength. More preferably, it is 35-55%. Still more preferably, it is 40-50%.

In a porous part, if a circumference part of communicating pores formed by causing pores to overlap with each other is comprised of the thickness of a particle of calcium phosphate, as a result, the surface area is preferably enlarged.

In the present invention, it is preferable that pores of a porous part are formed by stirring and bubbling of a slurry because a pore diameter can be controlled as a whole while maintaining characteristic pore shape that if the slurry is prepared by foaming, pores are substantially globular and communicating pores are numerous and large.

The biomember of the present invention, in particular, the porous part is preferably comprised of hydroxyapatite having an excellent strength among calcium phosphates sintered bodies, and the purity is preferably not less than 98%, particularly 100%. Similarly, the compact part is preferably comprised of hydroxyapatite.

If active materials are attached to the pore inner surface of a porous part of such a biomember, it is preferable to further accelerate regeneration of the bone. The active materials are preferably combinations comprising at least one or two of cell adhesion promoting material, cell proliferation promoting material, osteogenesis promoting material, bone absorption inhibiting material and vascularization promoting material.

Further, if at least one of osteogenic cells, automyelocytes, homogeneous myelocytes, fetal myelocytes and undifferentiated stem cells is introduced into a pore of a porous part of the biomember, it is also preferable to accelerate regeneration of the bone.

Similarly, if at least one of osteogenic cells to which a gene of an active factor is introduced, automyelocytes to which a gene of an active factor is introduced, homogeneous myelocytes to which a gene of an active factor is introduced, fetal myelocytes to which a gene of an active factor is introduced and undifferentiated stem cells to which a gene of an active factor is introduced is preferably introduced into a pore of a porous part of the biomember, it is also preferable to accelerate regeneration of the bone. In case where various cells or various cells to which genes are introduced are introduced into the pore of such a porous part, various active materials can be used together.

When drugs are stored into a pore of a porous substance, it becomes sustained-release preparations, for example, the sustained release amount of the drugs can be controlled by adjusting the surface proportion of a compact part, and the drugs have directional property in elution.

Since the biomember of the present invention has the above-mentioned characteristics, it can be applied to all sorts of cell culture carriers in vitro, and of course, used in preparing a bone for a graft in vitro.

### BRIEF DESCRIPTION OF THE DRAWINGS

Fig. 1 is a photomicrograph enlarging a cross section of a biomember of an embodiment of the present invention;
Fig. 2 is a diagram showing main pores in Fig. 1;
Fig. 3 is a diagram showing pores having a size larger than a mean pore diameter in Fig. 1;
Fig. 4 is a diagram showing pores and communicating pores in Figs. 1 and 3;
Fig. 5 is an enlarged photograph in which resins are embedded in pores;
Fig. 6 is an enlarged photograph in which resins are embedded in pores;
Fig. 7 is a diagram showing a biomember buried in vivo;
Fig. 8 is a diagram showing a biomember buried in vivo, respectively;
Fig. 9 is a diagram showing a biomember coated by active materials;
Fig. 10 is a diagram showing a biomember coated by active materials and buried in vivo;
Fig. 11 is a diagram of a biomember which active materials are coated, cells are introduced into and is cultured in an artificial environment;
Fig. 12 is an enlarged photograph showing a cross section of the biomember presenting the other embodiment;
Fig. 13 is a graph showing the state of a pore distribution in the other embodiment having a mean pore diameter of 300 µ m;
Fig. 14 is a graph showing the state of a pore distribution in the other embodiment having a mean pore diameter of 190 µ m;
Figs. 15 to 23 show the other various embodiments of the present invention;
Fig. 24 is a photomicrograph enlarging a cross section of the biomember in Fig. 1;
Fig. 25 is a photomicrograph further enlarging a cross section of the biomember of Fig. 24; and
Fig. 26 is a photomicrograph further enlarging a cross section of the biomember of Fig. 25.

### DETAILED DESCRIPTION OF THE EMBODIMENTS

Hereinafter, embodiments of the present invention will be described in detail with reference to the drawings.

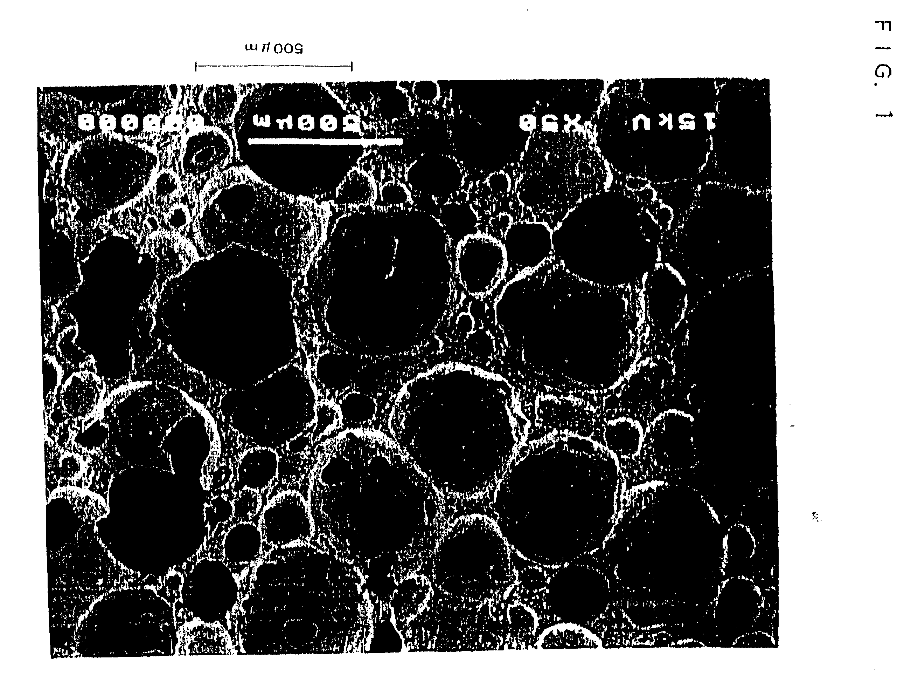

### Embodiments of Figs. 1 - 14

The biomember according to an embodiment of the present invention is comprised of a porous calcium phosphates sintered body, has no bio-harmfulness, and has relatively the strength even if it is a porous substance. What the porosity is not less than 55% and not more than 85% can enlarge a surface area of pores while maintaining the strength, and is very suitable for the biomember. Preferably, it is not less than 65% and not more than 85%.

In addition, what a pore diameter is not less than 50 µ m and not more than 800 µ m on the average is used. In up to 50 µ m is used, cell infiltration is difficult, and in more than 800 µ m, strength deterioration and decrease of a pore surface area occur. Preferably, a mean pore diameter is not less than 100 µm and not more than 600 µm, and more preferably, not less than 100 µm and not more than 350 µm.

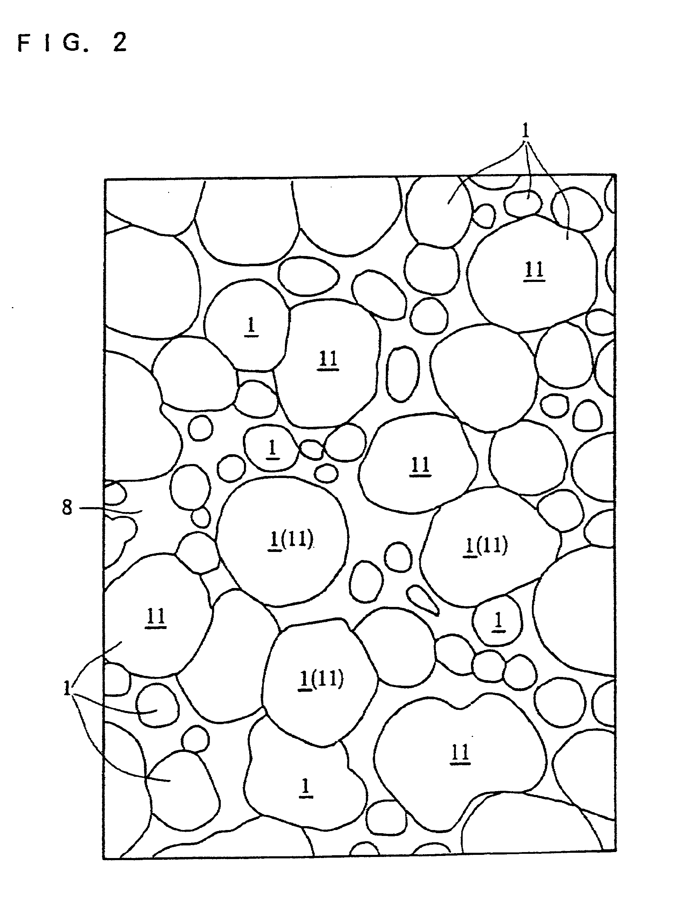

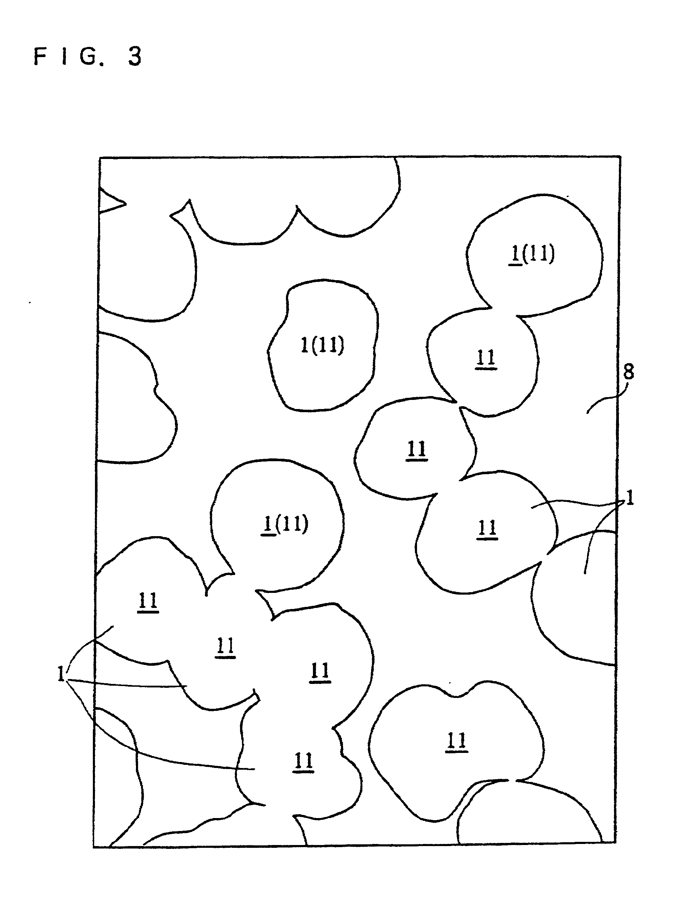

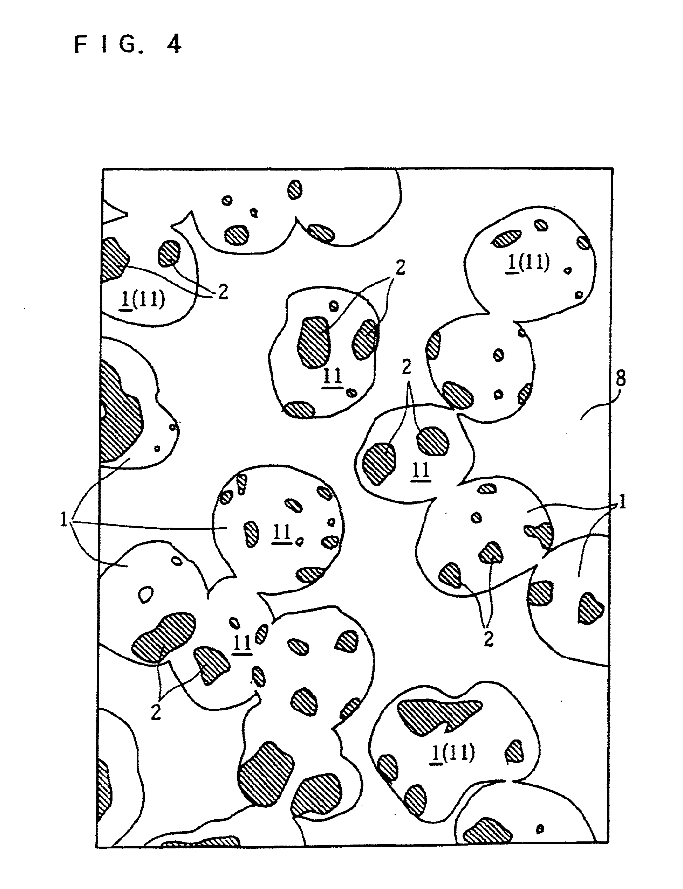

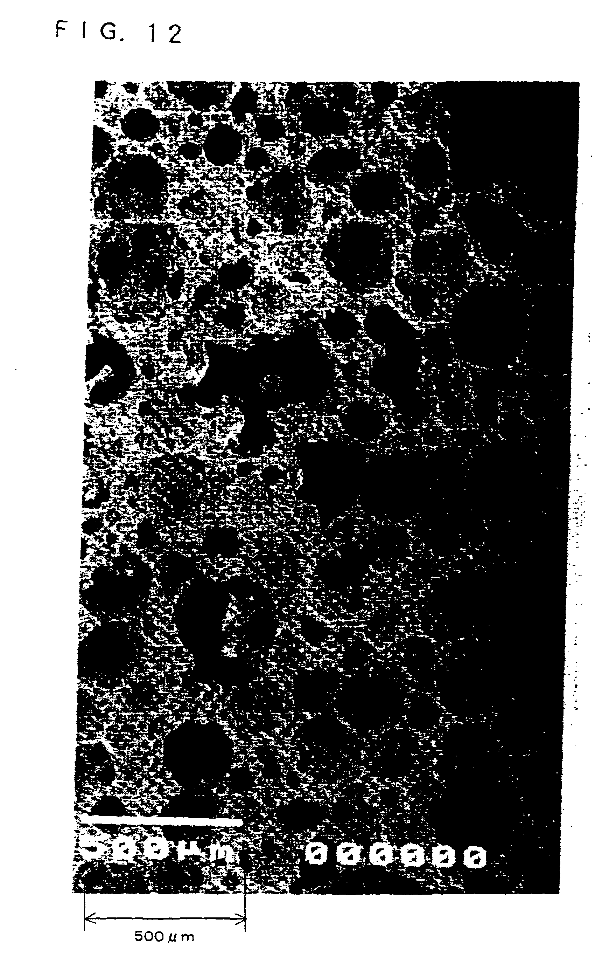

For example, a microphotograph of a cross section of a biomember comprising 100% hydroxyapatite and having a mean pore diameter of 300 µ m is shown in Fig. 1, and the one having a mean pore diameter of 150 µ m is shown in Fig. 12. Also, in Fig. 2, main pores 1 existing in the cross section in Fig. 1 are plotted, and in Fig. 3, only pores 11 having a size larger than the mean pore diameter in Fig. 2 are plotted. And, in Fig. 4, in the inner surface of the pore 11 in Fig. 3, opened parts (communicating pores 2) that communicate with other pores 1 are plotted two-dimensionally by hatching. Further, in Fig. 2 - Fig. 4, numeral 8 represents hydroxyapatite.

As known clearly from these drawings, the biomember of the present invention has a number of pores 1, and the pores 1 are formed in a substantially globular shape as a whole. The globular pore 1 has no directional property, and is easy to maintain the strength. Also, the pores 1 are formed in a substantially globular shape, but when communicating pores 2 are formed in an interface by coming into contact with adjacent pores 1, a flat configuration becomes the same configuration as an outline when two circles are overlapped partially.

Using such configuration is in order to enlarge a surface area. Even after sintering, an edge (open pore) remains sharply on the circumference of communicating pores 2, which are an interface of pores 1, 1 of the biomember. But, the edge may be removed a little by etching etc. so as to easily flow body fluid such as blood etc.

Further, in the pores 1, pores 11 having a size larger than the above-mentioned mean pore diameter have at least three communicating pores 2 having a diameter of not less than 5 µ m, on the average - they have communicating pores 2 having a diameter of not less than 5 µ m in the ratio of at least three points on the average - and therefore, body fluid is impregnated everywhere. In addition, what a diameter of one communicating pore 2 is 5 µ m "on the average" etc. defines the diameter in case of converting a transverse section configuration of the communicating pore 2 into a complete circle of the same transverse section volume. Also, since a pore having at least three communicating pores 2 on the average has at least one communicating pore 2 having a diameter of not less than 25 µ m, on the average, cells are easy to infiltrate into pores 11 in addition to the body fluid.

Generally, since human cell has a size close to 10 µm and an erythrocyte of an adult is also 8-9 µm in diameter, if there are communicating pores 2 of 25 µm, it is enough that oxygen or nutrients spread uniformly and cells penetrate. By having such large communicating pores 2 and communicating with many pores 11 three-dimensionally, body fluid circulates better in the whole biomember, and a cell easily infiltrates into a core part of the biomember. Additionally, since the circulation is enhanced remarkably by a communicating pore 2 of not less than 40 µm, such setting is preferable.

Preferably, pores 11 having a size larger than the mean pore diameter have at least four communicating pores 2 having a diameter of not less than 5 µm, on the average, and among them, it is preferable to have at least one communicating pore 2 having a diameter of not less than 50 µm, on the average. More preferably, pores 11 having a size larger than the mean pore diameter have at least six communicating pores 2 having a diameter of not less than 10 µm, on the average, and among them, by having at least two communicating pores 2 having a diameter of not less than 50 µ m, on the average, circulation of body fluids into pores 11 becomes active. In addition, it is particularly preferable that the foregoing (at least two points on the average) communicating pore 2 having a diameter of not less than 50 µm has a diameter of not less than 80 µm.

However, it is preferable that pores 11 having the foregoing large communicating pore 2 and a size larger than the mean pore diameter are opened as the communicating pore 2 in the ratio of not more than 50% of a pore inner surface area (in other words, the total opening area of the communicating pore 2 which is possessed by the pore 11 having a size larger than the mean pore diameter occupies the proportion of not more than 50% of the pore surface area on the average). If the pore inner surface area to be lost as communicating pores 2 is larger than 50%, a surface area to attach cells becomes too small. Further, it also affects strength. Preferably, it is not more than 40%.

In satisfying the foregoing conditions, the pore inner surface should be wet in detail by water or blood in order that body fluid or cells infiltrate easily. In this regard, as mentioned in the above, because the present invention uses pores 11 of the specific state and it has a homogeneous structure through inner part, a sintered body which is processed if necessary and then washed and dried can soak water by a capillary phenomenon in a dry state without carrying pretreatment, for example, if a part is immersed in water (pure water). Also, it has characteristics that it is possible to flow through an inner part to reach a bottom part by dropping water. It is particularly preferable that blood (whole blood) is the same as water.

In addition, the "in a dry state" means no treatment such as coating of surfactants or previous wetting by priming etc., and it is possible to use without pretreatment for a living body However, this expression is not the meaning of limiting a practical using method. Also, various limitations relating to pores 11 having a size larger than the mean pore diameter are because pores 11 having a size larger than approximately the mean pore diameter substantially affect extremely the effects etc.

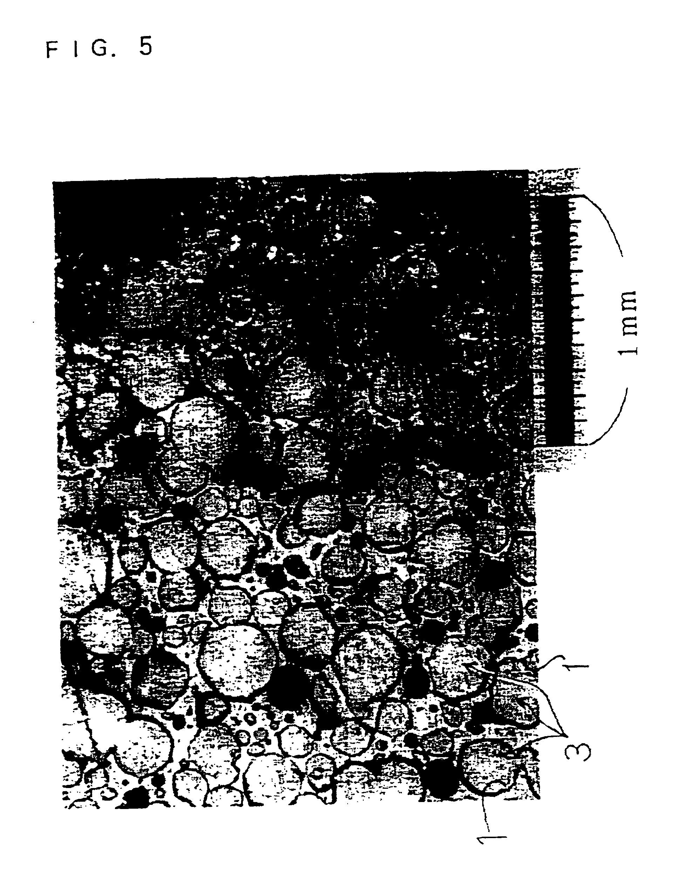

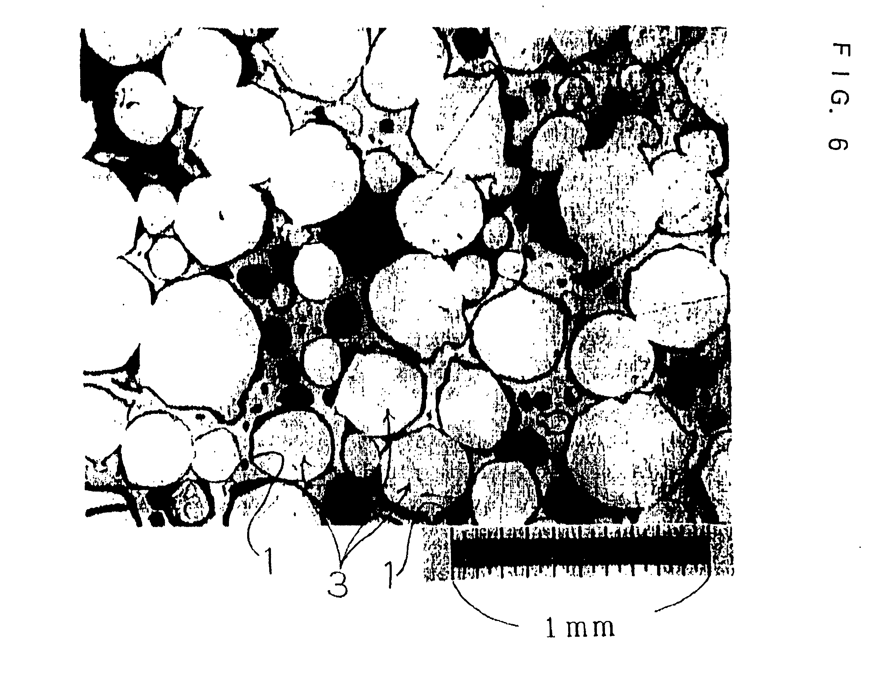

By the way, the mean pore diameter described in the present invention can be measured, for example, by resin embedding (filling pore 1 with resin). And, the 50% volume pore diameter i.e., a diameter of a pore when the value is exactly 50% of total pores by integrating volumes in a large pore (or a small pore) is referred to as the mean pore diameter. Additionally, the state that pore 1 is filled with resin 3 is shown in Fig. 5 and Fig. 6. As a result of measurement, Fig. 5 shows that the mean pore diameter is 1.90 µ m, and Fig. 6 shows that the mean pore diameter is 300 µm.

Also, in any plain cross section, when observing the state of pore 1 in plane aspect, pores 11 having a size larger than the mean pore diameter is preferably not less than 25% and not more than 60% of a flat area. That is, a sum of area of pores 11 shown in Fig. 3 is not less than 25% and not more than 60% of a total area in Fig. 3. In up to 25%, since pore parts become smaller, cell infiltration becomes difficult, and in more than 60%, strength is liable to be weak. More preferably, it is not less than 35% and not more than 55%. Still more preferably, it is not less than 40% and not more than 50%.

In the biomember of the present invention, because a cylinder part of the communicating pore 2 formed causing a pore 11 to overlap with a pore 11 having a size larger than the mean pore diameter, that is, the above-mentioned edge (open pore) is formed sharply and thin in about the thickness of one calcium phosphate particle to enlarge surface area, it is believed that the thin formed part (edge) is substituted promptly for a bone.

Such characteristics of pores 1 (11) can be obtained from foaming by stirring slurry raw material, and then, drying and sintering. Since the pore is not formed by baking and taking-out using globular particles of polystyrene etc., pressure for forming is not needed, there is no directional property that pores 1 (11) become plain, etc., and simultaneously, compared with that a contact point of polystyrene is opened, in the present invention, the communicating pore 2 becomes remarkably large, and simultaneously, a surface area can be enlarged. Additionally, it is preferable that in calcium phosphate particles of slurry raw materials, a mean particle diameter is of the submicron order (i.e., not less than 0.1 µm and up to 1 µm), and it is preferable that the maximum particle diameter is also of the submicron order.

In addition, for example, Japanese Patent Laid-Open No. hei 10-167853 describes a porous body that a circumferential part of a communicating pore is formed sharply, but because it is made by baking and taking-out, the communicating pore is small not more than 10 µm, and it is difficult that a cell penetrates.

The biomember of the present invention can be processed into a desired shape, and at the same time, fixed in vivo, and thus, blood etc. infiltrate the inner part and oxygen or nutrients spread uniformly. If body fluid such as blood etc. circulate, cells begin to be attached to the inner wall surface of the member. The biomember of the present invention has a large surface area, and has many chances that cells are attached. Also, since pores 11 having a size larger than the mean pore diameter have open pore parts of not less than 25 µ m, blood etc. are easy to infiltrate inwardly. By having the open pore parts to be not less than 40 µ m, blood etc. are easy to remarkably infiltrate inwardly.

Since pores 1 are connected with each other, an inner part of the member begins to be substituted for the bone promptly. Since blood spread fast and uniformly as a whole mainly through pores 11 having a size larger than the mean pore diameter, blood etc. spread uniformly in small pores 1 having a size less than the mean pore diameter as similar as large pores 11.

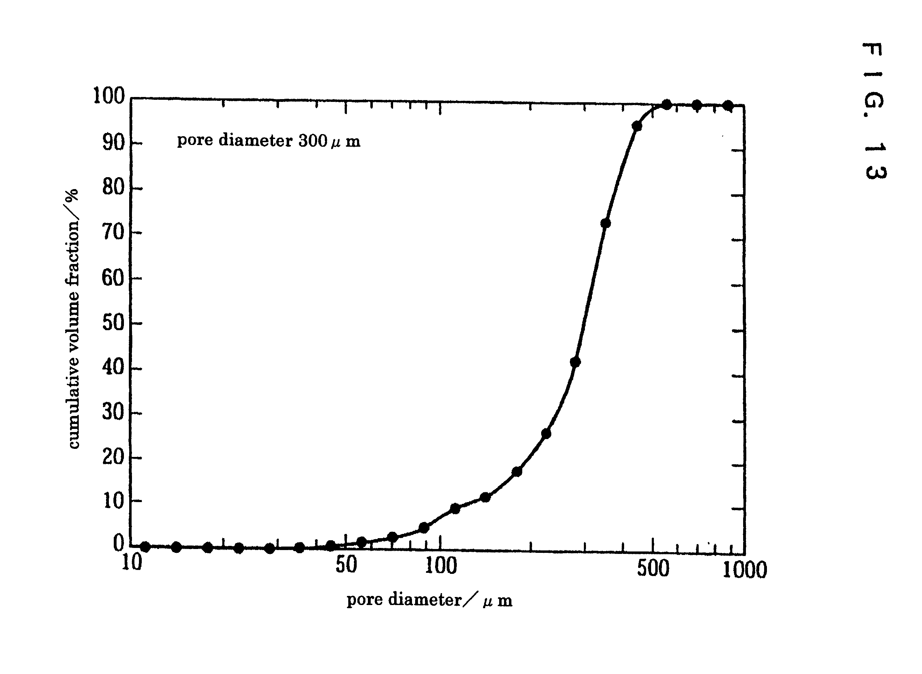

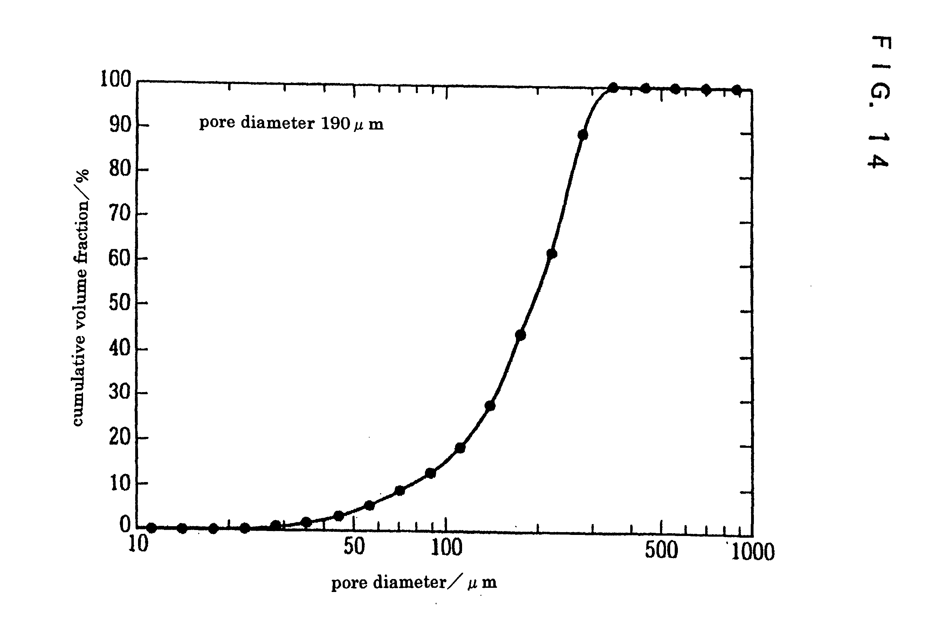

In the biomember of the present invention, the maximum pore diameter is preferably within three times of the mean pore diameter. Locally, too large pore are not preferable in strength and cell adhesion. Preferably, it is within two times thereof. The graph of Fig. 13 is a cumulative volume fraction of a sintered body pore having the mean pore diameter of 300 µm. The graph of Fig. 14 is also a cumulative volume fraction of sintered body pore having the mean pore diameter of 190 µm. In all, not less than 50% of the total pores are included within a range of ± 30% of the mean pore diameter.

As such, in the present invention, not less than 50% of the total pores are preferably included within a range of ± 30% of the mean pore diameter. Also, the cumulative volume fraction of pore of not more than 20 µ m is preferably approximately 0, and further, even if a skeleton surface of a calcium phosphate porous body is observed microscopically, there hardly exist pores, and it is preferable that there exists only uneyenness by rounding of calcium phosphate particles.

The biomember is preferably comprised of hydroxyapatite particularly excellent in strength. Its purity is preferably at least 98%, and in particular, 100% is good. Such a member can be obtained from, for example, Toshiba Ceramics Co., Ltd.

Further, as such, in the present invention, various materials suitable for forming bones are coated in the pore inner surface by using the features that blood etc. are easy to infiltrate into inner part as a whole, that a communicating pore is large, and that a surface area is large. The coated materials are active materials such as cell adhesion promoting material, cell proliferation promoting material, osteogenesis promoting material, bone absorption inhibiting material, vascularization promoting material and so forth, or cells and cells in which a genetic recombination is performed.

They are in a liquid phase or cultured in a broth, and infiltrate everywhere by using the features of the biomember of the present invention. In general, they easily spread uniformly as a whole by immersion, but in case where cells are big or have a high viscosity by a cell culture, they can be sucked by applying a negative pressure on a side where the biomember is present.

In any case, by using a biomember having both excellent penetrating property and excellent adhesive property to the surface with regard to the whole, they can spread uniformly and consistently to a core part even in a thick member, while it was impossible in the conventional product.

Hydroxyapatite has strong adsorptivity, and in particular, protein or anchorage dependent cells are easily adsorbed on the surface of hydroxyapatite. In addition, even in case where it is difficult to be adsorbed by simple contact, cell adhesive protein such as laminin etc. or heparin etc. is previously added to hydroxyapatite, and then, it is more preferable that an active material or a cell to be attached is added.

The active material may be cell adhesive promoting material. The cell adhesive promoting material is used as a term including "a constitutional element of an extracellular matrix" and "adhesive molecules". The constitutional element of the extracellular matrix includes (1) a basal membrane, (2) fibrillar protein of collagen, elastin, etc., (3) cell adhesive glycoprotein of fibronectin, laminin, vitronectin, etc., (4) complex carbohydrates of glycosamidglycan etc. including heparin, hyaluronic acid, chondroitin sulfuric acid, but it is not limited thereto.

Further, the adhesive molecule is the same meaning as a cell adhesive molecule, adhesive factor, and adhesive protein, and includes E-selectin, P-selectin, ICAM-1 (= intercellular adhesion molecule-1), VCAM-1, CD-18, CD-44, etc., but it is not limited thereto.

Further, the active material may be cell proliferation promoting material. The cell proliferation promoting material means material exhibiting physiological activities such as growth, division, differentiation of cell and functional promotion, etc., and includes "a proliferation factor" and "a mitogenic factor". The proliferation factor is the same meaning as a growth factor, and includes TGF-β superfamily, HGF (=hepatocyte growth factor), etc., but it is not limited thereto. Further, mitogenic factor is the same meaning as mitogen, and includes lectins of concanavalin A etc., but it is not limited thereto.

Further, the active material may be osteogenesis promoting material. The osteogenesis promoting material (it is called osteogenic factor) is BMP family, TGF-β superfamily, SAMP8 and so forth. Also, the active material may be bone absorption inhibiting material. The bone absorption inhibiting material is caldeclin etc.

Or, the active material may be vascularization promoting material. The vascularization promoting material is VEGF (=vascular endothelial growth factor), PDGF (=platelet-derived growth factor), b-FGF (b-fibroblastic growth factor), VEGF receptor (Flt-1 (VEGFR-1), Flk-1 (VEGFR-2), Flt-4 (VEGFR-3)), angiopoitin family receptor, trehalose 6, 6'-dimycolate (=TDM, TIE2/TEK, TIE1) and so forth.

And, the active material may be a combination comprising at least two of cell adhesive promoting material, cell proliferation promoting material, osteogenesis promoting material, bone adsorption inhibiting material and vascularization promoting material. The active material is a liquid phase, and is formed easily as a film phase on pore surface by immersing a biomember or bring it into contact. Then, it may be dried and can be preserved for a long time by refrigeration or cold preservation. Of course, it may be used instantly without preservation.

Further, an osteogenic cell may be introduced into the pore 1 (11). The osteogenic cell may be an osteoblast, and may include a chondrocyte. Just as it is taken out from a living body, or the osteogenic cell is previously introduced into the biomember described in claims 1 to 10 in vivo or in vitro, as it is, or the osteogenic cell to be re-cultured is applied to a patient. Thereby, after the operation, recovery that should be made for several days or weeks in vivo by the patient for himself/herself will be beforehand performed in vitro.

Or, an automyelocyte may be introduced into the pore 1 (11). In particular, when using the automyelocyte, of course, there is neither rejection reaction nor concern of infection by diseases such as hepatitis etc. But, in case of the aged etc., when a cell itself of the patient has no vitality, excellent effects cannot be expected.

Further, a homogeneous myelocyte may be introduced into the pore 1 (11). When using the homogeneous myelocyte to which another person's cell is introduced, the cell of the operated region can have vitality though the cell of the person himself/herself has no vitality.

Further, a fetal myelocyte may be introduced into the pore 1 (11). Or, an undifferentiated stem cell may be introduced into the pore. The undifferentiated stem cell is, for example, ES cell (=Embryonic stem cell), EG cell (=Embryonic germ cell) and so forth.

Further, if an osteogenic cell, automyelocyte, homogeneous myelocyte, fetal myelocyte or undifferentiated stem cell is introduced into the member to which at least one or two of the foregoing active materials are attached, the actions of the cell can be more active.

Or, an osteogenic cell to which gene of active factor is introduced may be introduced into the pore 1 (11). The active factor enables activation of growth, division, differentiation of a cell and functional promotion, etc. such as TGF- β , BMP, HGF, EGF and so on, and a suitable one is chosen if necessary. Since they are incorporated into a cell in a genetic level, the osteogenic cell gets to act actively.

Further, an automyelocyte to which a gene of an active factor is introduced may be introduced into the pore 1 (11). As mentioned in the above, in case of the automyelocyte, there is neither rejection reaction nor concern of infection, and even in the aged, his/her own cell gets to act actively by genetic recombination.

Further, a homogeneous myelocyte to which a gene of an active factor is introduced may be introduced into the pore 1 (11). A fetal myelocyte to which a gene of an active factor is introduced may be introduced into the pore 1 (11). Or, an undifferentiated stem cell to which a gene of an active factor is introduced may be introduced into the pore 1 (11).

Further, an automyelocyte to which a gene of an active factor is introduced, homogeneous myelocyte to which a gene of an active factor is introduced, fetal myelocyte to which a gene of an active factor is introduced or undifferentiated stem cell to which a gene of an active factor is introduced may be introduced into the member to which at least one or two of the foregoing active materials are attached. The combinations thereof exhibit the greatest effect in the present invention.

Further, by filling or coating drugs to pore the inner surface of the biomember of claims 1 to 10, for example, by means of immersion, impregnation, aspiration and so on, sustained release preparations can be made. At this time, after completion of sustained release, a calcium phosphates sintered body may be taken out in vitro, and if possible, may be regenerated as a bone.

Examples will be illustrated with reference to the embodiments of Figs. 1 to 14 of the present invention.

### Example 1:

Prisms (10 x 20 x 40 mm) for a biomember comprising a sintered body of 100% of apatite, having a porosity of 75% and a mean pore diameter having the same pore shape as Fig. 1 and Fig. 12 is 300 *µ* m and 150 µ m were prepared, respectively; and 1 cc of blood dropped thereto, and then, it was absorbed immediately as if it droped on a sponge.

### Example 2:

Four types of cylinders comprising biomembers with φ 10x6 mm (diameter 10 mm, length 6 mm) made of hydroxyapatite of the present invention, having a porosity of 75% and a mean pore diameter of 150 µm, 300 µm and 600 µm, respectively, and a similar hydroxyapatite biomember (comparative example) with φ 10x6 mm made by A Pharmaceutical Company, having the porosity of 50% and the mean pore diameter of 100 µm were prepared; and about 200 µl of a culture solution (this is a liquid comprising amino acid or plasma containing many kinds of cytokines, and its viscosity is close to that of water) dropped to each of them, and then, all of three types of the biomembers of the present invention absorbed the entire solution like a sponge. And, the biomember of A Pharmaceutical Company (of comparative example) did not absorb the culture solution at all.

### Example 3:

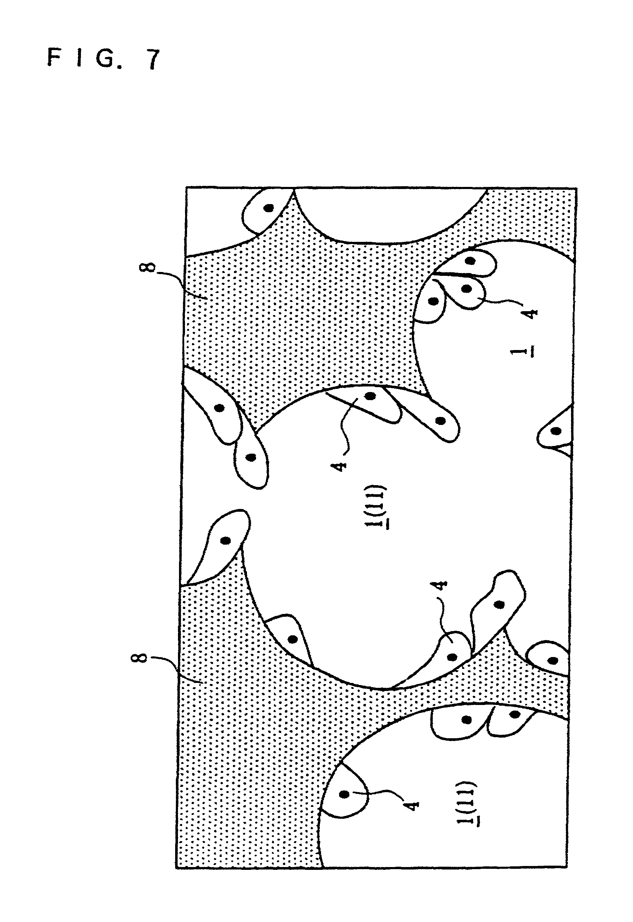

On the biomember φ 10x6 mm made of hydroxyapatite of the present invention, having a mean pore diameter of 300 µ m, a cell suspension containing osteoblasts dropped, and then, the biomember absorbed it immediately like a sponge. Thereafter, under the conditions of 37°C and 5% CO₂, it was cultured for 2 days, and as the result of observing an inner part of the cylinder, adhesion of numberless cells was ascertained on the inner part. Fig. 7 shows the state that cells 4 are attached into the pore 1 (11).

### Example 4:

Three types of biomembers made of hydroxyapatite of the present invention, having a porosity of 75% and a mean pore diameter of 150 µ m, 300 µ m, 600 µ m were prepared as cylinders of φ 6x15 mm, respectively.

They were embedded in a thighbone of a rabbit, and were taken out 1 week, 3 weeks, 6 weeks after operation; they were further fixed by formalin, and decalcificated, and then, stained by hematoxylin - eosin. With respect to the thus treated biomembers, tissue infiltration into hydroxyapatite and a bone neogenesis state were observed by an optical microscope.

The results are shown below as (1) - (3).
(1) 1 week after operation, a granulation tissue was ascertained in all of the pore inner parts of three types of the biomembers having a mean pore diameter of 150 µm, 300 µm, and 600 µm, and blood vessels were also partially observed. A bone neogenesis was a few detected in a surface layer of hydroxyapatite.
(2) 3 weeks after operation, a bone neogenesis was shown up to an extremely deep core part (central part) of the cylinder of φ 6 mm as if it sticks along the surface of the pores, and as the result of measuring the surface area of the bone neogenesis part, with respect to the mean pore diameters, 300 µ m was somewhat larger than 600 µ m, and 150 µ m larger than 300 µ m. In this time, with respect to ones having a larger pore diameter, blood vessels could be more clearly ascertained. Further, there existed a number of myelocytes on the central part of such a pore.
(3) 6 weeks after operation, in addition to the bone neogenesis of (2), myelocytes were observed in the pores having all of the pore diameters. It is believed that they had hematogenous functions and became close to the state of a bone marrow before filling up hydroxyapatite. Also, in this step, as a result of measuring the strength of hydroxyapatite, it was enhanced to the extent of about two times than the case before filling it up.

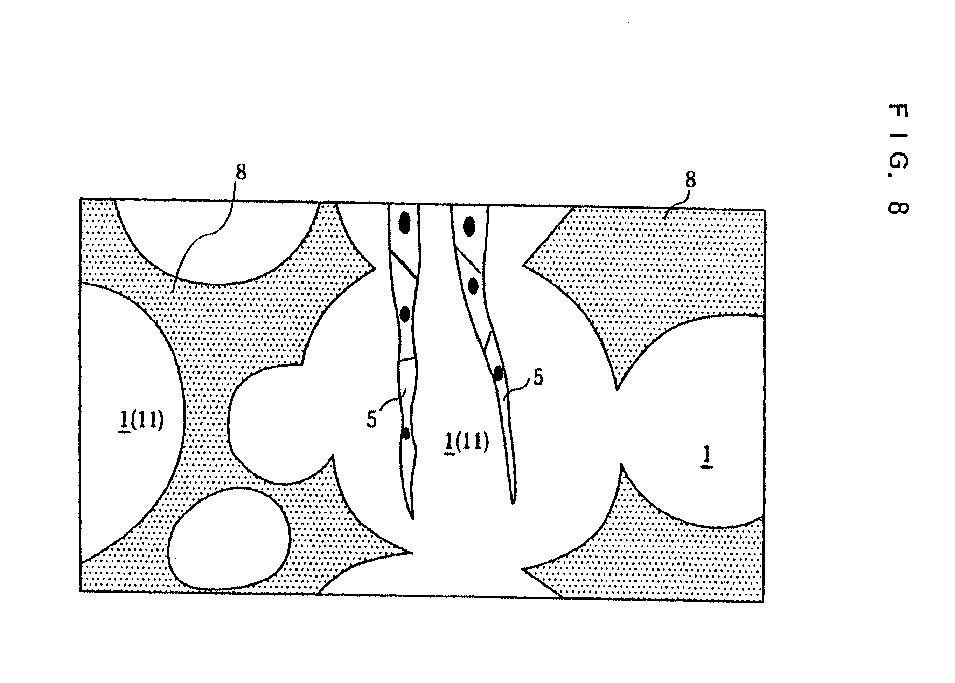

Fig. 8 shows the state that a small blood vessel 5 begins to be formed in a pore 1 (11) (but, the state of a cell was omitted). myelocytes 9 can be generated in a pore 1 (11)

### Comparative example 1 relative to Example 4:

A member with a diameter of 6 mm and a length 15 mm, having the above-mentioned characteristics (A Pharmaceutleal Company's) was embedded in a thigh bone of a rabbit, and as the result of observing 3 weeks, 6 weeks after operation, adhesion with a cowl bone was ascertained on the surface of apatite, but tissue infiltration into an inner part was not shown at all.

From the results of the above examples 1 to 4 and comparative example 1, the mean pore diameter of about 100 - 600 µ m is useful for cell fixation or bone regeneration, and above all, it is preferable that a pore has a comparative small mean pore diameter of not less than 100 µm and not more than 350 µm. In particular, the mean pore diameter of not less than 120 µm and not more than 220 µm is superior.

### Example 5:

Two kinds of cylinders (a), (b) with φ 10x6 mm; a cylinder (a) "no growth factor" and a cylinder (b) "adding 3 µ g/block of VEGF vascular endothelial growth factor", having a porosity of 75% and a pore diameter of 300 µm having the same pore configuration as Fig. 1 were grafted to a latissimus dorsi muscle subfascia of a mouse. Three weeks after grafting, they were taken out, and then, a tissue in apatite was observed. The results are shown below as (4) and (5).
(4) In the cylinder with no growth factor, cell infiltration was merely about 1 mm in the surface layer of apatite.
(5) In the cylinder adding VEGF, cell infiltration was observed up to an apatite core part (this means a cell infiltrated 3 mm - 4 mm and more in the surface layer).

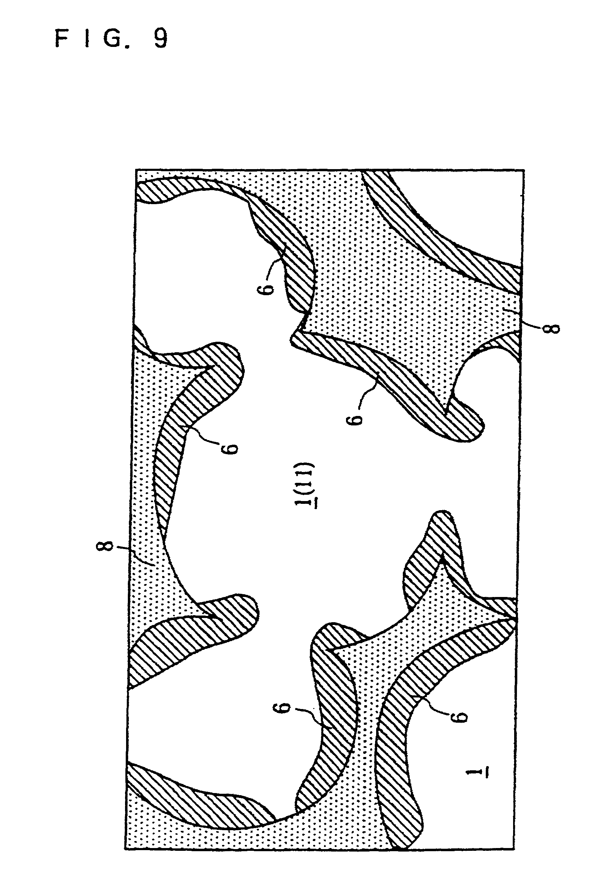

Fig. 9 shows the state that an active material 6 was attached to a pore 1 (11). The active material 6 is illustrated exaggeratedly to easily understand the attached state, but in practical, even if protein etc, are coated, the thickness is sometimes hardly shown. In addition, if it is collagen etc., any degree of thickness can be ascertained.

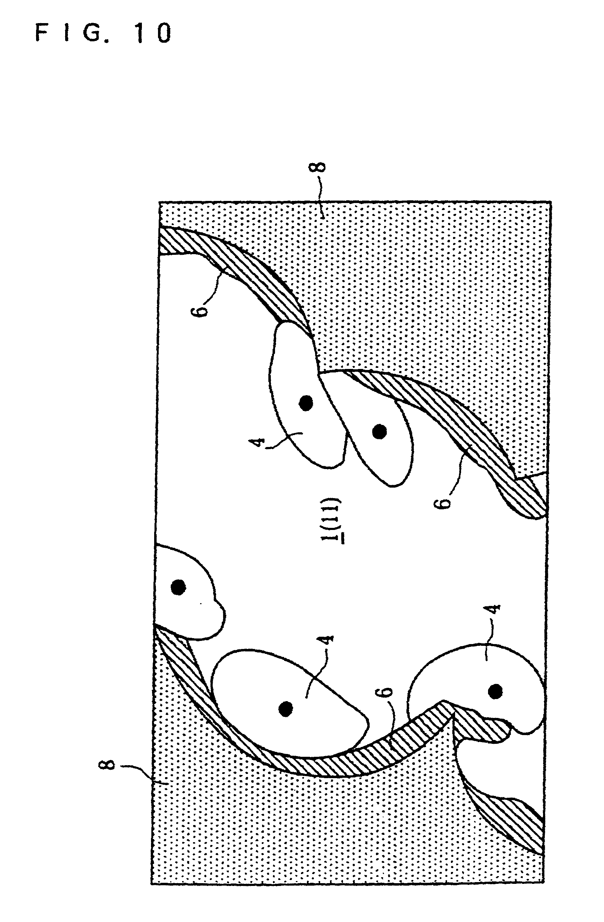

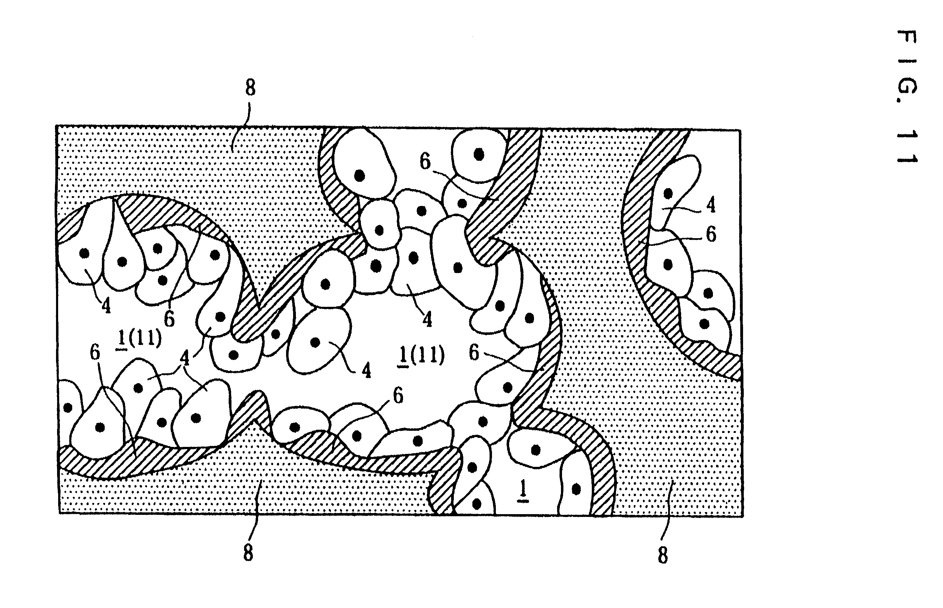

Fig. 10 shows the state that in the member of Fig. 9, a cell 4 was attached to the active material by an animal experiment. Fig. 11 shows the state that cell 4 is introduced into the member in Fig. 9, and many cells 4 were increased in a pore 1 (11) by culture.

Further, the number etc. of cells in Figs. 7 to 11 are indicated so as to understand relatively the respective characteristics, and the actual state is not accurately expressed. Substantially, the cell is visible slightly smaller, and the number thereof becomes also more numerous.

Fig. 13 shows a pore distribution of the biomember in Fig. 1, and Fig. 14 shows a pore distribution of the biomember with a mean pore diameter of 190 µm, having the same pore as that of Fig. 1.

The graphs of Fig. 13 and Fig. 14 represent the cumulative volume fractions of sintered body pores having a mean pore diameter of 300 µ m and 190 µ m. In the present invention, like the graphs, at least 50% of total pores volume is preferably included within a range of ± 30% of the mean pore diameter. Also, the cumulative volume fraction of pores of not more than 20 µm is preferably approximately 0, and additionally, it is preferable that in observing microscopically a skeleton surface of a calcium phosphate porous body, there hardly exist pores, and there exists only unevenness caused by rounding of calcium phosphate particle.

It is preferable that pores are concentrated around the mean pore diameter such that a cumulative volume fraction included in the range of a pore diameter having a cumulative volume fraction of 50% to a 30% larger pore diameter than the pore diameter, that is, in the range of a pore diameter of 300 µ m and 390 µ m in the case of the mean pore diameter of 300 µ m, is not less than 25%; and it is particularly preferable if not less than 30%. That is because these pores are the most important ones to obtain the effects of the present invention.

By the way, in general, for example, when an old man of about 70 years suffers a fracture, depending on the fracture portion and fracture degree, it usually takes several weeks to one year to recovery. In particular, in fracture of a thighbone, it takes more time, and if a sick period becomes longer, a bedridden tendency is higher.

In this regard, if the biomember of the present invention is used, the period in which an injury is healed will be decreased by half, estimated by a cell culture state, and by using genetic recombination technology, it is more reduced, and further, a patient who suffers inconvenience in a daily life due to deterioration of a bone density can be expect to recover. Further, it can be sufficiently applied to treat osteoporosis.

In this manner, in the biomember of the present invention, because a substantially globular pore 1 (11) is formed and the shape of the globular pore is maintained in the area other than a communicating pore 2, the surface area per unit volume is remarkably large while the communicating pore 2 is secured, body fluid is penetrated into an inner part by a capillary phenomenon, and the proportion of contact with blood is high, and therefore, much more cells 4 can be easily attached.

Further, since an active material 6 can be spread over the whole, and is easily attached on the surface, it is easily prepared to attach various active materials 6 or introduce cells, and furthermore, thereafter culture is also easy, and, remarkable recovery after operation will be possible by applying it to the patient.

The biomember of the present invention can be not only used when a bone is defected but also applied to sustained-release preparations which are caused to indwell in vivo to release drugs for a long period. Also, a compact biomember may be arranged properly in order to enhance the strength in a core part or a part of the outer surface of a biomember. Even so, because circulation of body fluids is possible only in a porous part of the surroundings, the effects of the present invention can be sufficiently expected. Further, the shape of the biomember of the present invention may be various, and of course, if necessary, may be granular.

Since it is comprised of a porous calcium phosphates sintered body, it has good compatibility with a living body, has no bio-harmfulness. And, since the porosity is set to be an appropriate value, the strength required for compensating the defected portion of a bone can be sufficiently secured. And, it can be properly processed into a necessary form, and furthermore, since a structure is homogeneous through an inner part, the member is of definite quality and of good reliability.

The pores 11 communicate with each other through a communicating pore 2, and therefore, the surface area per unit volume can become larger, body fluid is penetrated into an inner part by an excellent capillary phenomenon, the proportion of contact with blood becomes high, much more cells 4 can be attached, and integration with a living body is performed promptly.

Also, in order to enhance the strength etc., even if a compact member is properly arranged to the core part, circulation of body fluid is possible only in a porous part of the surroundings, and thus, extensive application can be made depending on symptoms.

Also, the biomember can be not only applied in the case where a bone is defected, but also applied to sustained-release preparations which are caused to indwell in vivo to release drugs for a long period. In this case, if integration with a living body is possible, re-operation for enucleation is not needed.

Because the mean pore diameter is set to be a more preferable value, a more excellent capillary phenomenon can be expected, and body fluid can be penetrated into an inner part more efficiently.

Because the diameter of the communicating pore 2 is set to be a more preferable value, circulation of body fluid into the pore 1 (11) becomes more active.

Because the ratio of the pore 11 having a size larger than the mean pore diameter occupied in a flat area is set to be a preferable value, infiltration of the cell 4 into the pore 1 (11) becomes easy, and simultaneously, the desired strength can be secured.

Because the ratio of the pore 11 having a size larger than the mean pore diameter occupied in a flat area is set to be a more preferable value, infiltration of the cell 4 into the pore 1 (11) becomes further easy, and simultaneously, the desired strength can be more properly secured.

Because water infiltrates into a core part by a capillary phenomenon, body fluid or cells can be impregnated to the core part.

Because blood infiltrates into a core part by a capillary phenomenon, body fluid or blood can be impregnated to the core part.

Because the thickness of a circumferential part of the communicating pore 2 becomes about the thickness of one calcium phosphate particle, the edge (open pore) is formed thin and sharply, and thus, the surface area becomes larger, and the thin part is easily substituted for a bone.

Because the pore 1 is formed from foaming by stirring of a slurry, the pore becomes substantially globular, and a diameter of a communicating pore becomes enlarged. Also, a circumferential part of the communicating pore 2 is formed thin and sharply, and the surface area becomes larger, and therefore, the thin part is easily substituted for a bone.

Because a calcium phosphates sintered body is comprised of hydroxyapatite 8, particularly, protein or anchorage dependent cells are easily attached on the surface.

Because an osteogenic cell is introduced into the pore 1, after operation, recovery that should be made by a patient himself/herself for several days or weeks in vivo will be beforehand performed in vitro, and therefore fast recovery can be expected.

Because an automyelocyte is introduced into the pore 1, there is neither rejection reaction, nor concern of infection by diseases of hepatitis etc.

Because a homogeneous myelocyte is introduced into the pore 1, cells in an operated region can have vitality even though the cells of the person himself/herself have no vitality.

Because a fetal myelocyte is introduced into the pore 1, actions of cells can be more active.

Because an undifferentiated stem cell is introduced into the pore 1, actions of cells can be more active.

Because an osteogenic cell to which a gene of an active factor is introduced is introduced into the pore 1, activation of growth, division and differentiation of cells, functional promotion, etc. becomes possible, and they are incorporated into cells in a genetic level, and therefore, osteogenic cells act actively.

Because an automyelocyte to which a gene of an active factor is introduced is introduced into the pore 1, there is no fear of rejection reaction and concern of infection, and, even in the aged, his/her own cells can be caused to act actively by genetic recombination.

Because a homogeneous myelocyte to which a gene of an active factor is introduced is introduced into the pore 1, cells in an operated region can have vitality even though the cells of the person himself/herself have no vitality.

Because a fetal myelocyte to which a gene of an active factor is introduced is introduced into the pore 1, actions of cells can be more active.

Because an undifferentiated stem cell to which a gene of an active factor is introduced is introduced into the pore 1, actions of cells can be more active.

Because the active material 6 is attached to the inner surface of the pore 1, cells are easily attached.

Because the active material 6 is cell adhesion promoting material, cells are easily adhered (adsorbed).

Because the active material 6 is cell proliferation promoting material, cell proliferation is promoted.

Because the active material 6 is osteogenesis promoting material, formation of a bone is promoted.

Because the active material 6 is bone absorption inhibiting material, absorption of a bone is suppressed.

Because the active material 6 is vascularization promoting material, vascularization is promoted.

Because the active materials 6 are combinations comprising at least two of cell adhesion promoting material, cell proliferation promoting material, osteogenesis promoting material, bone absorption inhibiting material and vascularization promoting material, cell adhesion or cell proliferation becomes active, formation of a bone is promoted, or absorption of a bone is suppressed, and also, vascularization is promoted.

Because an osteogenic cell is introduced, after operation, recovery that should be made by a patient himself/herself for several days or weeks in vivo will be beforehand performed in vitro, and fast recovery can be expected.

Because an automyelocyte is introduced, there is no fear of rejection reaction and concern of infection by diseases of hepatitis etc.

Because a homogeneous myelocyte is introduced, cells in an operated region can have vitality even though the cells of the person himself/herself have no vitality.

Because a fetal myelocyte is introduced into the pore 1, actions of cells can be more active.

Because an undifferentiated stem cell is introduced into the pore 1, actions of cells can be more active.

Because an osteogenic cell to which a gene of an active factor is introduced is introduced into the pore 1, activation of growth, division and differentiation of cells, functional promotion, etc. become possible, and they are incorporated into cells in a genetic level, and therefore, osteogenic cells act actively.

Because an automyelocyte to which a gene of an active factor is introduced is introduced into the pore 1, there is no fear of rejection reaction and concern of infection, even in the aged, his/her own cells can be caused to act actively by genetic recombination.

Because a homogeneous myelocyte to which a gene of an active factor is introduced is introduced into the pore 1, cells in an operated region can have vitality even though the cell of the person himself/herself have no vitality.

Because a fetal myelocyte to which a gene of an active factor is introduced is introduced into the pore 1, actions of cells can be more active.

Because an undifferentiated stem cell to which a gene of an active factor is introduced is introduced into the pore 1, actions of cells can be more active.

It can be used as sustained-release preparations.

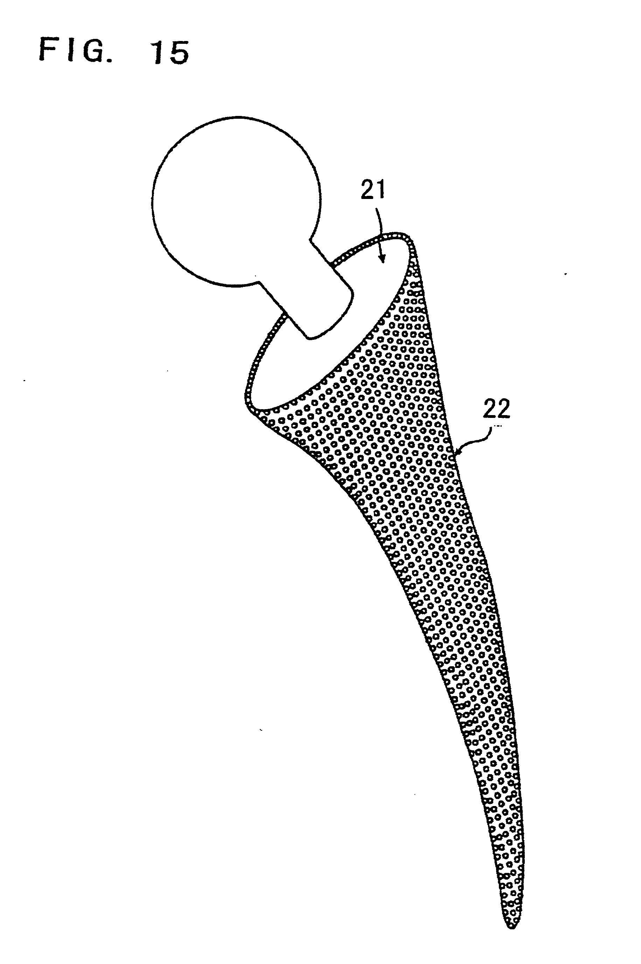

### Embodiment of Fig. 15

In inserting a stem part of an artificial joint into a bone, an osteoepiphysis is removed, a hole for inserting the stem is formed by a drill etc., and then the stem is inserted to be fixed. The stem is generally made of titanium alloy etc., and is difficult to be compatible with a bone. For that reason, it takes time to be fixed to the bone, and meantime, since deviation is occurred whenever load is applied, a patient sometimes feels a pain.

In the embodiments of the present invention, a calcium phosphates porous body having a specific pore shape is arranged on the outer surface of a portion where an artificial joint is inserted to a bone lobe. Thereby, it is easy to be compatible with the bone due to the specific pore shape, and accelerate fixation to mitigate pains at an early stage.

When a porous body to form the outer surface of the biomember of the present invention is practically formed in a block shape and inserted into a bone lobe, regeneration of a bone begins in the region of the porous body, and the aspect is taken white in Roentgen, and it can be identified only in 3 weeks while it takes about three months in the conventional porous body.

A compact member 21 is processed into a desired shape to be fixed in vivo. A porous member 22 is infiltrated by blood etc., and oxygen or nutrients are spread sufficiently If body fluid such as blood, circulates, cells are easy to be attached to the inner wall surface of the member.

The biomember of the present invention has a large surface area, and thus, there are many chances that cells are attached. Also, since large pores having a size larger than the mean pore diameter have an opening portion of at least 25 µm, blood etc. can easily infiltrate thereto. Because pores are connected with each other, any region of the inner part of the member begins to be quickly substituted for a bone.

Since blood is spread fast over the whole, centering around pores having a size larger than the mean pore diameter, blood etc. are also spread uniformly as the same as the large pores even in small pores having a size smaller than the mean pore diameter.

In the porous member of the biomember of the present invention, the maximum pore diameter is preferably within three times of the mean pore diameter. Locally too large pores are not preferable in strength and cell adhesion. Preferably, it is within two times thereof.

The graphs of Fig. 13 and Fig. 14 accord with the embodiment of Fig. 15, too.

The biomember of the present invention is preferably comprised of hydroxyapatite particularly excellent in strength among calcium phosphates sintered bodies. The purity is preferably at least 98%, and in particular, 100% is preferable.

Further, as such, in the present invention, various materials suitable to form bones can be coated in the pore inner surface by using such features that blood etc are easily infiltrated as a whole and the surface area is large.

As the coated materials, there are active materials such as cell adhesion promoting material, cell proliferation promoting material, osteogenesis promoting material, bone absorption inhibiting material, vascularization promoting material ,etc., cells and genetic recombinant cells and so forth.

They are in a liquid phase or cultured in a broth, and infiltrate everywhere by using the features of the biomember of the present invention. In general, they are easily panetrated over the whole by immersion, but when cells become big or have a high viscosity owing to cell culture, they can be sucked by applying a negative pressure on the side where the biomember is present. In any case, by using the biomember of the present invention having both excellent penetrating property over the whole and excellent adhesive property to the surface, they can be penetrated uniformly and consistently to a core part even in a thick member, which was impossible in the conventional product.

Examples will be illustrated below with reference to the embodiment of Fig. 15 of the present invention.

### Example 6:

A hydroxyapatite film was formed on the outer surface of a titanium alloy rod having a diameter of 10 mm, a length of 100 mm and a porosity of 0 by spray coating, and a granular porous apatite layer having a mean particle diameter of 1 mm, a porosity of 75%, and a mean pore diameter of 150 µm was installed thereon by a thickness of 1.5 mm, and then, bonded by heating.

### Example 7:

A granular porous apatite layer having a mean particle diameter of 2 mm, a porosity of 75%, and a mean pore diameter of 300 µm was installed on the outer surface of a titanium alloy rod having a diameter of 10 mm, a length of 100 mm and a porosity of 0 through an adhesive by a thickness of 2.5 mm, and then, bonded.

### Example 8:

An apatite film was formed on the outer surface of an alumina rod having a diameter of 10 mm, a length of 100 mm and a porosity of 10% by spray coating, and a granular porous apatite layer having a mean particle diameter of 1 mm, a porosity of 75%, and a mean pore diameter of 150 µm was installed thereon by a thickness of 1.5 mm through a slurry containing apatite, and then, bonded by heating.

### Example 9:

A foamed slurry containing apatite was coated on the outer surface of an apatite rod having a diameter of 10 mm, a length of 100 mm and a porosity of 0, and sintered. Then a porous member 22 having a porosity of 75% and a mean pore diameter of 150 µ m was installed by a thickness of 3 mm.

### Example 10:

A foamed slurry containing apatite was coated on the outer surface of an alumina column having a size of 10 x 10 x 100 mm and a porosity of 5%, and a previously sintered porous member 22 having a porosity of 75% and a mean pore diameter of 150 µ m was bonded on one side of the column by a thickness of 2 mm, and then, heated.

In any one of the examples 6-10, porous granules were fixed firmly to the compact body. Also, pores of the porous body maintained a specific shape. When blood dropped on these porous members 22,it was penetrated extensively in the whole of the porous members22.

In the biomember of the present invention, the respective pores 3 of the porous member 22 forming a part or the whole of the outer surface have a relatively equal size and they communicate with each other. Particularly, in pores having a size larger than the mean pore diameter, because communicating pores are large, infiltration of blood or cells is easy, regeneration of a bone begins at an early stage in the porous member 22 comprised of calcium phosphate, and a bone can be compatible with the compact member 21.

Accordingly, while a communicating pore is secured, a surface area per unit volume is remarkably large, body fluid is penetrated into an inner part by a capillary phenomenon, the proportion of contact with blood is high, and therefore, much more cells can be easily attached.

Further, active material etc. can be penetrated over the whole, and be easily attached to the surface, and therefore, it is easily prepared to attach various active materials or introduce cells, and furthermore, thereafter, culture is easy and remarkable recovery after operation will be possible by using it to a patient. When such a porous member 22 is arranged on the outer surface of the compact member 21, compatibleness with a bone is fast and integrated at an early stage.

The compact member 21 is not readily compatible only in itself because of a gap or deviation even though it is buried into a bone, but because the porous member 22 having a specific pore shape is installed thereon, compatibleness with a bone is remarkably accelerated. Further, the strength is maintained by the compact member 21 (compared with the biomember comprised wholly of porous substances).

That is, blood or cells are easily impregnated into pores. Also, regeneration of a bone can be promoted. Further, the member has no directional property and is easy to maintain the strength. Further, it is possible to enlarge the area where cells are attached.

Further, because a part or the whole of the outer surface of the compact member 21 is comprised of the porous member 22, it is possible to enlarge the surface area of pores while maintaining the strength and to promptly regenerate a bone. Also, body fluid is impregnated everywhere. Also, in addition to body fluid, cells also infiltrate easily into pores. Also, a penetrating amount of oxygen, nutrients, cells, etc. are preferably markedly increased. Further, circulation of body fluid becomes better in the whole biomember, and cells are easy to infiltrate into a core part of the biomember.

Further, circulation of body fluid into a pore becomes active. Also, pressure forming is not needed, there is no directional property that pores become flat, and simultaneously, compared with a member in which a contact point of urethane is opened, a communicating pore becomes remarkably large, and simultaneously, a surface area can be enlarged.

In a dry state, it is possible to wet the whole by dropping water and blood. Also, even in a dry state without pretreatment, for example, once a part is immersed into water, water can be soaked by a capillary phenomenon. Also, it has characteristics such that it is possible to flow through an inner part to reach a bottom part by dropping water. Also, it is possible to be used for a living body without pretreatment.

Because a calcium phosphates sintered body is different from metal or other kinds of ceramics in thermal expansion coefficient etc., fusion or bonding is often difficult, but the fusion or bonding can be easily performed by installing an intermediate layer.

Further, the fusion or bonding of various materials can be easily performed by the intermediate layer. The integration of a bone with the biomember can be made earlier.

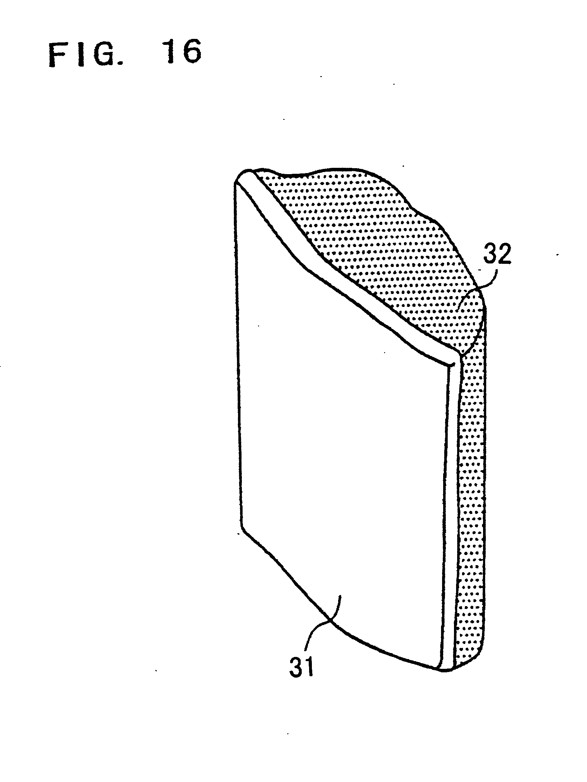

### Embodiments of Figs. 16 - 23

Fig. 16 shows a biomember used for the portion which should be excised due to disease or injury.

For example, the portion which should be excised due to disease or injury is removed previously by operation, and depending on the conditions of the defected portion, a biomemeber having an appropriate size and shape is prepared. At this time, the shape and size of the biomember are selected so that the outer surface of a bone becomes a compact part 31, and are processed, if necessary. In insertion, a porous part 32 is placed on an inner part of a bone, and the compact part 31 is placed in such a way as to form the same side as the outer surface of the bone.

At that time, a splint is held to the compact part 31 to reduce stroke, and is put little by little while tapping by a plastic hammer etc. The surface of the porous part 32 may be slightly broken by contact with a bone, but because broken powder functions to fill up the rest of a space, and on the contrary, it is more preferable than the remaining of the rest of a space between the bone and the biomember. The compact part 31 resists strike-installation, and thus, it is difficult that the strike side is crushed. Also, it is good for the member to always maintain the shape as a whole and form the outer surface of the bone. The compact part 31 may be cracked by stroke, however, since there is no case that it is crushed to pieces, it does not affect the recovery after operation.

It is preferable that the compact part 31 is a compact body of apatite.

According to the degree of load in burying or using a biomember, if necessary, metal of titanium etc. or ceramics of alumina etc. may be used, or metal of titanium etc. or ceramics of alumina etc. which are coated with apatite may be used for the compact part 31 in replace of the compact body of apatite.

The porous part 32 of the biomember is formed by foaming a slurry, fixing bubbles and sintering. At this time, it is preferable that calcium phosphate particles of slurry raw material have a mean particle diameter of a submicron order (i.e., not less than 0.1 µm and up to 1 µm) and the maximum diameter is also preferably of a submicron order.

Further, it may be prepared by using two kinds of slurrys different in foaming amount. Also, it may be possible that the compact part 31 is previously formed by a common method for forming fine ceramics and a foamed slurry flows thereon to be fixed. Also, the compact part 31 and porous part 32 formed separately may be bonded before or after sintering and fixed. In this case, an intermediate layer having intermediate property may be disposed between the compact part 31 and the porous part 32.

A pore is formed in substantially globular shape, but in case where communicating pores are formed in the interface by connecting with adjacent pores, a flat configuration becomes the similar configuration to an outline when two circles are caused to overlap partially with each other. Using such a shape is intended to enlarge a surface area. A communicating pore which is the boundary of pores of the biomember of the present invention is remarkably large compared with those by baking and taking-out of beads, and simultaneously, even after sintering, an edge remains sharply on a circumference of the communicating pore.

The edge may be removed slightly by etching etc. so as to easily flow body fluid such as blood etc.

The pore diameter of the present invention can be measured, for example, by resin embedding. And, the 50% volume pore diameter (i.e., a diameter of a pore when the value is exactly 50% of the total pores by integrating volumes in a large pore or a small pore) is referred to as the mean pore diameter.

In the member of the present invention, since pores are connected with each other through a large communicating pore, an inner part of the member begins to be substituted promptly for a bone. Since blood is penetrated fast over the whole, centering around pores having a size larger than the mean pore diameter, blood etc. are penetrated similarly to large pores in small pores having a size smaller than the mean pore diameter.

In the porous part 32 of the biomember of the present invention, the maximum pore diameter is preferably within three times of the mean pore diameter. A locally too large pore is not preferable in strength and cell adhesion. Preferably, it is within two times thereof.

The graphs of Fig. 13 and Fig. 14 also accord with the embodiments of Figs. 16 - 23. As the graphs, in the porous part 32, it is preferable that not less than 50% of total pores are included within a range of ±30% of the mean pore diameter. Also, a cumulative volume fraction of a pore of not more than 20 µ m is preferably approximately 0, and further, even if a skeleton surface of the porous part 32 (calcium phosphate porous body) is observed microscopically, there hardly exist pores, and it is preferable that there exists only unevenness due to rounding of calcium phosphate particles.

Further, as such, according to the present invention, various materials etc. suitable for forming bones can be coated in the pore inner surface by using such features that the surface area is large so that blood etc. are easy to infiltrate into an inner part as a whole.

The coated materials are at least one of the active materials such as cell adhesion promoting material, cell proliferation promoting material, osteogenesis promoting material, bone absorption inhibiting material, vascularization promoting material and so forth, cells and genetic recombinant cells.

They are in a liquid phase or cultured in a broth, and infiltrate everywhere by using the features of the biomember of the present invention. In general, they are easily penetrated over the whole by immersion, but when cells become big or have a high viscosity owing to cell culture, they can be sucked by applying a negative pressure on a side where the biomember is present. In any case, by using the biomember of the present invention having both excellent penetrating property over the whole and excellent adhesive property to the surface, they can be penetrated uniformly and consistently to a core part even in a thick member which was impossible in the conventional product.

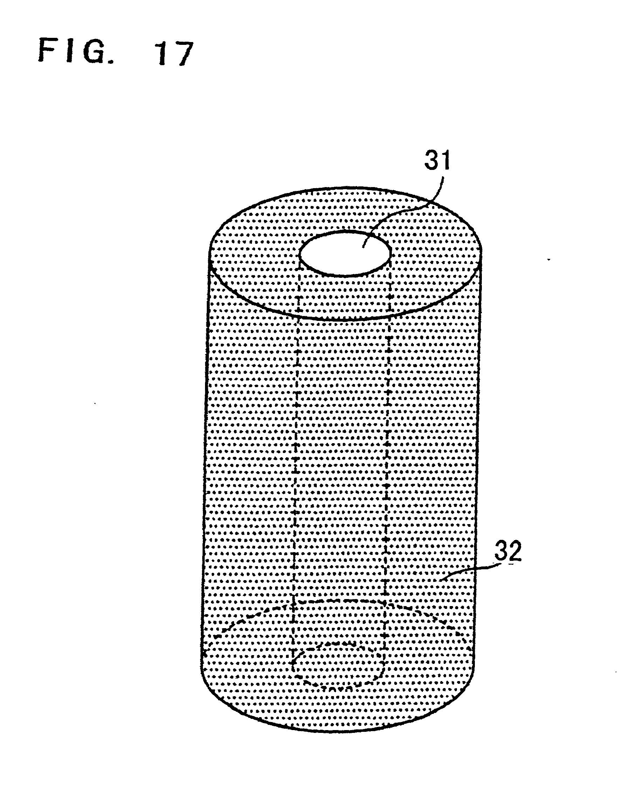

Fig. 17 shows the other embodiment. A porous part 32 is a cylindrical body, and a compact part 31 becomes a cylinder held and installed in an inner part of the porous part 32. In this case, because the compact part 31 is formed in the inner part, in case where heavy load is applied, it is suitable to support load applied from the exterior. That is, while the outer surface of the porous part 32 in the outer surface is substituted for and regenerated to a bone and strength is occurred, it can protect the porous part 32 and maintain the strength of the biomember.

Since the compact part 31 is formed in the inner part of the porous part 32, the compact part 31 plays a role in supporting load and protecting the porous part 32. In this case, it is preferable that the compact part 31 is a compact body of apatite.

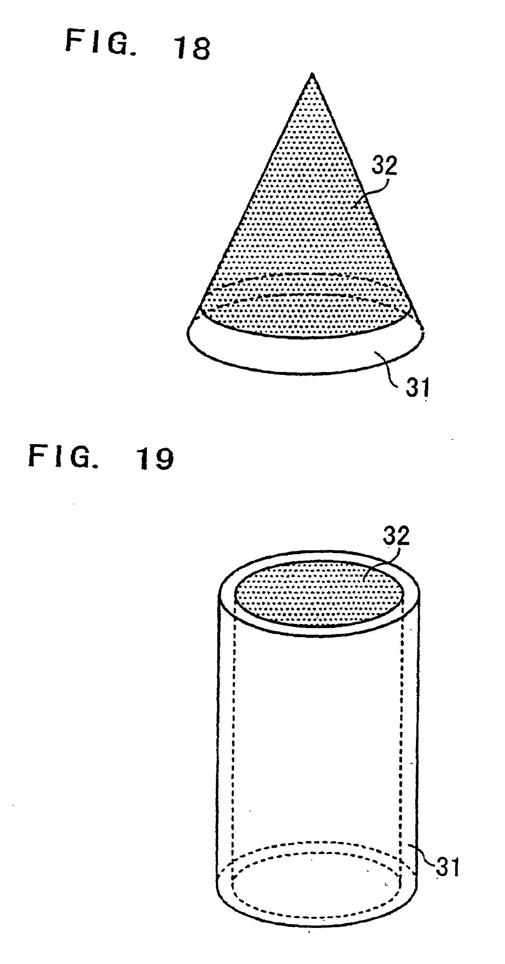

Fig. 18 also shows still other embodiment. The whole shape is a circular cone, the bottom surface is a compact part 31, and an apex side is a porous part 32. The shape is often used in actual operation. And, the biomember can be struck and installed by tapping the compact part 31.

Fig. 19 also shows still other embodiment. A compact part 31 is a cylindrical body, and a porous part 32 becomes a cylinder installed in an inner part of the cylindrical body in a receivable shape. For example, a porous cylinder is inserted into a compact cylindrical body by tapping, and integrated. In this case, for instance, an intermediate part of a bone of rhabdome such as a femur, humerus, etc. is preferably comprised of only the biomember of the present invention.

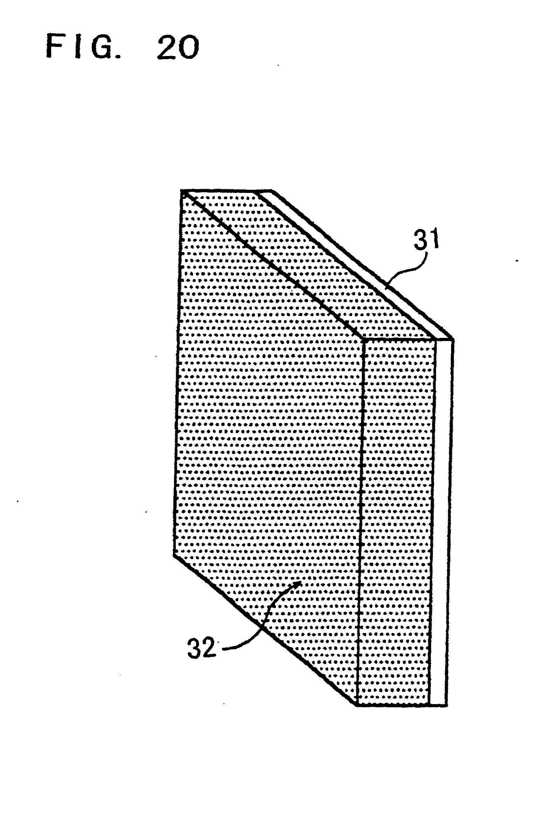

Fig. 20 also shows still other embodiment. The whole shape is cuboid, and only one side of outer surfaces becomes a compact part 31. For example, it is preferably suitable to bury the biomember of the present invention into a part of a large bone.

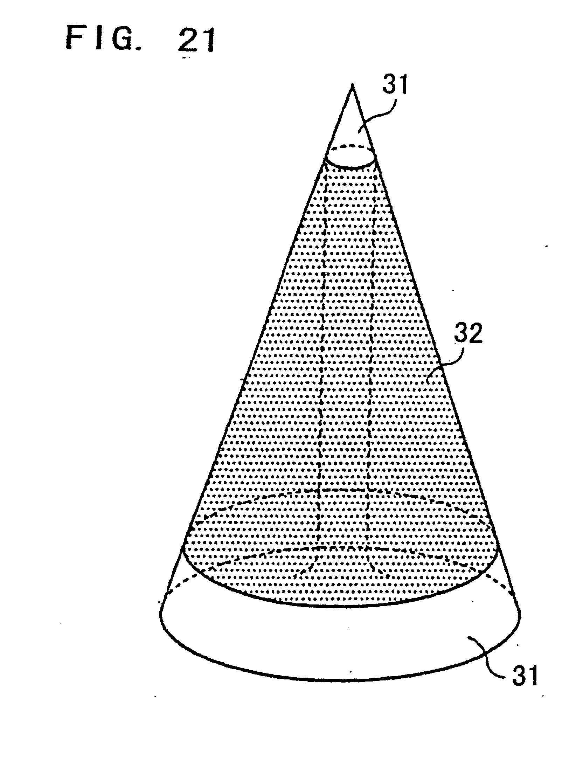

Fig. 21 also shows still other embodiment. A compact part 31 is formed in a part of the outer surface and an inner part. Specifically, the whole shape is a circular cone, and the bottom surface is the compact part 31. And, in a direction toward the inner part from a stroke part (the bottom), the compact part 31 is vertically installed.

By the above-mentioned constitutions, for example, when it is buried into a bone, the compact part 31 can be inserted by tapping. Also, while the porous part 32 of outer surface is substituted for a bone, the strength of the biomember can be maintained. Also, toward the inner part from the stroke part, the compact part 31 is vertically installed, and thus, stroke power is efficiently transmitted to the inner part.

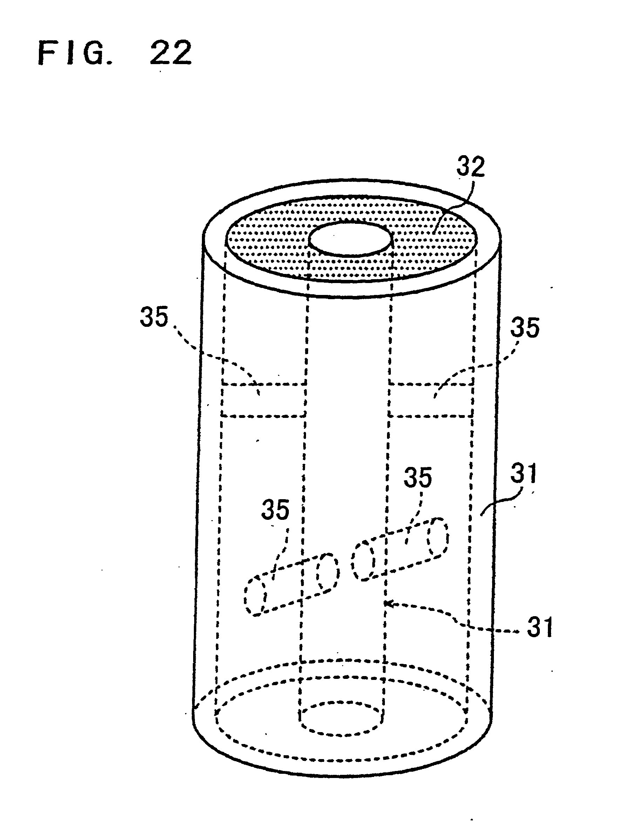

Fig. 22 also shows still other embodiment. The whole shape is columnar, and a compact part 31 is installed in an outercurved side and innercurved side of a porous part 32 having a substantially cylindrical shape. And, the compact part 31 in the outercurved and innercurved sides of the porous part 32 having a substantially cylindrical shape is connected to a branched part 35 installed in a radial direction at predetermined height.

By the construction of Fig. 22, for example, when it is buried into a bone, of the compact part 31, the compact part 31 in the innercurved side of the porous part 32 having a substantially cylindrical shape is preferably inserted by tapping. Also, while the porous part 32 is substituted for a bone, the strength of the biomember is preferably maintained.

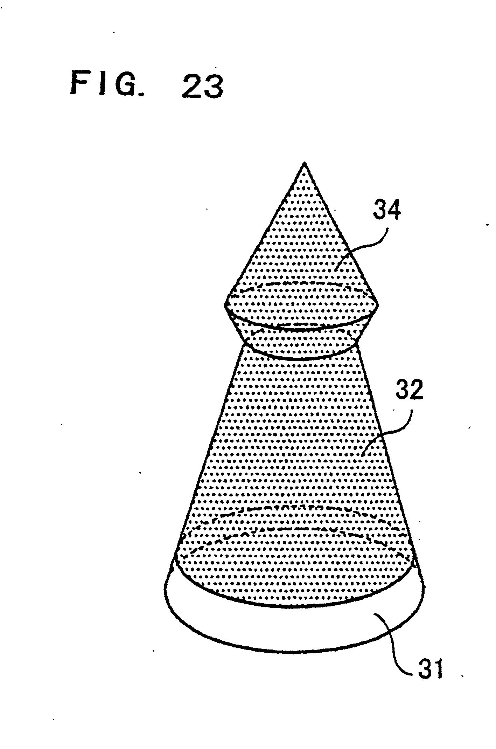

Fig. 23 also shows still other embodiment. The whole shape is substantially conical, and an ampulla 34 of a porous substance is installed in the vicinity of an apex of the conical body. When it is inserted to bone, a part of the ampulla 34 is trimmed. Thereby, the rest of space can be filled.

Examples will be illustrated with reference to the embodiments of Figs. 16 - 23 of the present invention.

### Example 11:

A biomember was prepared to be a cone comprised of apatite 100% and having approximately a diameter of 20 mm and a height of 26 mm from the bottom surface and a porous part (porous part 32) having a porosity of 75% and a pore shape like Fig. 1 and a mean pore diameter of 300 µ m, and compact part (compact part 31) having a porosity of 10%. The compact part was used for the bottom only by a thickness of 3 mm, and the other part was the porous part.

This was inserted while lightly tapping the compact body by a plastic hammer into a hole of a diameter of 20 mm and a height of 25 mm of the bottom surface correctly formed in metal. The compact part could be completely inserted without smash. As a result of offtake after insertion, in the lateral side of the cone, a part of the porous substance part was crushed to powders, and the lateral side of the cone was suitable for the hole of the metal mold. 0.5 ml of blood dropped on the taken-out conical body, and it was entirely absorbed immediately in the porous part as if it drops on a sponge.

### Comparative example 2 relative to example 11:

Similarly to example 11, a cone (a diameter of the bottom surface is approximately 20 mm) without a compact part and comprised of only a porous part was inserted to a metal mold by tapping, and therefore, it was the same that a slant part was crushed partially to powders, but a part of stroke part was also crushed to powders. 0.5 ml of blood dropped on the taken-out conical body, it was entirely absorbed immediately in the porous part as if it drops on sponge.

### Example 12:

A cylindrical body of compact apatite having a diameter of 25 mm, an inner diameter of 20 mm and a height of 37.5 mm was prepared, and an apatite slurry was filled therein for forming a porous part, dried and sintered. A completed sintered body was a cylindrical body having a porosity of 75% of porous part, a diameter of 20 mm and a height of 20 mm. The sintered body endured up to 200 MPa against compression from the upper and lower sides.

### Example 13:

A cylindrical body of a compact apatite sintered body having a diameter of 20 mm, an inner diameter of 14 mm and a height of 20 mm was prepared, and a cylindrical body comprising a porous sintered body made of an apatite slurry and having a diameter of 14.1 mm and a height of 20 mm was inserted therein. The sintered body endured up to 280 MPa against compression from the upper and lower sides.

### Comparative example 3 relative to examples 12 and 13:

A cylindrical body of a porous apatite sintered body having a diameter of 20 mm, a height of 30 mm and s porosity of 75% was prepared. The sintered body endured up to 17 MPa against compression from the upper and lower sides.

As such, in the biomember of the present invention, substantially globular pores are formed in the porous part 32, meanwhile, a number of large communicating pores are secured, and therefore, a surface area per unit volume is remarkably large, body fluid is penetrated into an inner part by a capillary phenomenon, the proportion of contact with blood is high, and thus, much more cells can be easily attached.

Further, because active materials can be penetrated over the whole and be easily attached on the surface, it is easy to prepare to attach various active materials and introduce cells, and thereafter, culture becomes easy, and recovery after operation becomes possible by using it to a patient.

The biomember of the present invention can be used when a bone is defected, and further, be applied to sustained-release preparations which release drugs for a long period by putting it in vivo. Also, though the compact biomember may be arranged properly in order to enhance the strength on core part of the biomember, circulation of body fluid is possible only in the porous part 32.

In the porous part 32, blood or cells are easily impregnated into a pore. Also, regeneration of a bone can be promoted. Further, since it has the compact part 31, it is easy to maintain the strength. Also, the surface area to which cells are attached can be enlarged. And, since it has both the porous part 32 and the compact part 31, it is possible to enlarge the surface area of pores while maintaining the strength and to regenerate the bone promptly.

In the porous part, body fluid is impregnated everywhere. Also, in addition to the body fluid, cells also infiltrate easily into pores. Also, the penetrating amounts of oxygen, nutrients, cells etc. are preferably quite increased. Further, circulation of body fluid becomes better in the whole biomember, and thus, cells easily infiltrate into a core part of the biomember.

Further, in a dry state, the porous part can wet the whole by dropping water and blood. Also, even in a dry state without pretreatment, for example, once a part is immersed into water, it can soak water by a capillary phenomenon. Further, it is possible to flow through an inner part to reach a bottom part by dropping water. Also, it can be used for a living body without pretreatment. Meanwhile, since the compact part is suppressed to have a lower porosity, the strength is enhanced. Also, it can sufficiently take the porous part 32 substituted for a bone. For example, bending strength becomes about 80-150 MPa, and therefore, heavy load is applied. Thereby, it can endure stroke and its handling becomes much easier in operation etc. Also, it becomes the strength enough to sustain load applied by a bone of a patient.

If it is prepared by a method of foaming a slurry, maintaining a pore shape characterized in that substantially globular communicating pores are numerous and large, it is easy to control a pore diameter.

Among calcium phosphates sintered bodies, because hydroxyapatite exhibits particularly excellent strength, heavy load may be applied. Likewise, it can endure stroke, and its handling becomes even easier in operation etc. Also, it becomes strength enough to sustain the load applied by a bone of a patient.

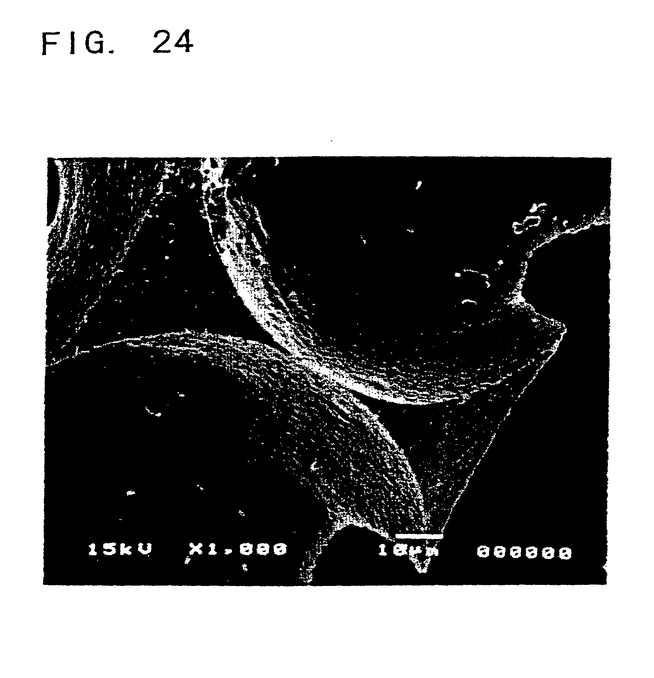

When it is used as sustained release preparations, by adjusting surface proportion of the compact part 31, a sustained-releasing amount of drugs can be controlled, and drugs have directional property in elution. Descriptions of Figs. 24-26 which are common to all of the above-mentioned embodiments
Fig. 24 is a photomicrograph enlarging a cross section of the biomember in Fig. 1.

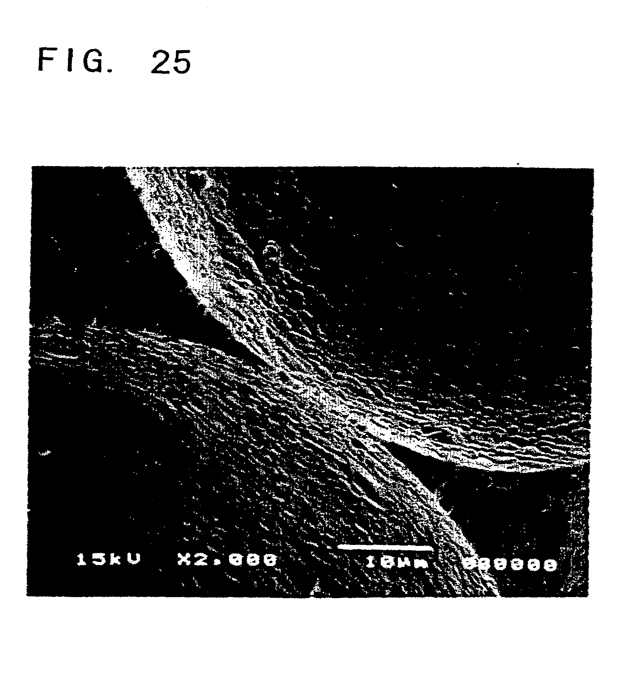

Fig. 25 is a photomicrograph further enlarging a cross section of the biomember in Fig. 24.

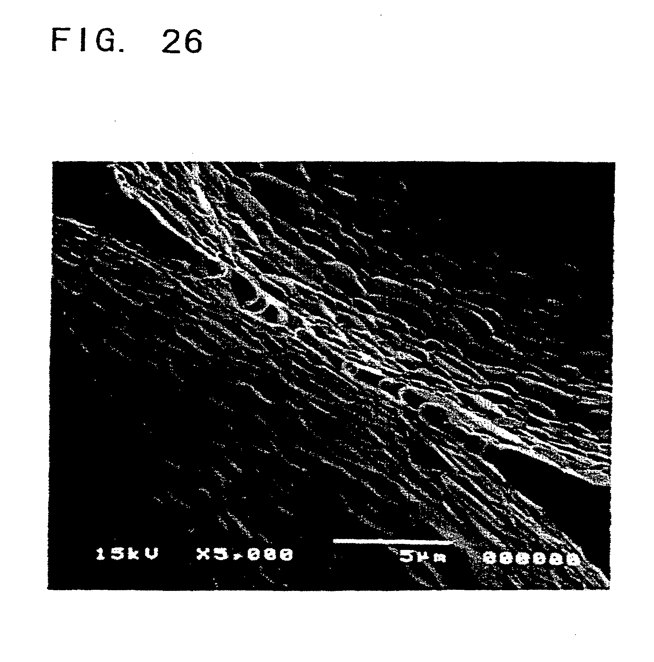

Fig. 26 is a photomicrograph further enlarging a cross section of the biomember in Fig. 25.

The present invention is not limited to the above-mentioned embodiments.

## Claims

1. A biomember for forming a bone in a living body, consisting of a porous body of a calcium phosphates sintered body comprising a number of substantially globular pores (1), and of a compact part, wherein the compact part has a porosity of not more than 15%, the porosity of the porous body is not less than 65% and not more than 85%, and simultaneously, the mean pore diameter of the globular pores is not less than 100 µm and not more than 600 µm, a globular pore (11) having a size larger than the mean pore diameter has at least four communicating opened parts (2) having a diameter of not less than 5 µm, on the average, and simultaneously, a globular pore (11) having at least the four communicating opened parts (2) has at least one communicating opened part (2) having a diameter of not less than 50 µm, on the average, and simultaneously, the total opening area of the communicating opened part (2) which is possessed by the globular pore (11) having a size larger than said mean pore diameter of the globular pores, does not occupy more than 40% of the surface area of said globular pore (11) on the average, and in a dry state, it is possible to wet the whole by dropping water and blood,
wherein micro particles of submicron (i.e. not less than 0.1 µm and up to 1µm) order are used as raw material, and in the porous body of the calcium phosphates sintered body the particle diameter is not more than 5 micron,
wherein the globular pores (1) of the porous body are formed as a foam obtained by stirring a slurry of the raw material,
wherein the porous body of calcium phosphates sintered body is hydroxyapatite (8),
wherein an osteogenic cell, automyelocyte, homogeneous myelocyte, fetal myelocyte, undifferentiated stem cell, osteogenic cell to which a gene of an active factor is introduced, automyelocyte to which a gene of an active factor is introduced, homogeneous myelocyte to which a gene of an active factor is introduced, fetal myelocyte to which a gene of an active factor is introduced, or undifferentiated stem cell to which a gene of an active factor is introduced is introduced into a globular pore (1), whereby embryonic cells are excluded from before-mentioned cells.

2. The biomember according to claim 1, wherein the globular pore (11) having a size larger than the mean pore diameter has at least six communicating opened parts (2) having a diameter of not less than 10 µm, on the average, and simultaneously, a globular pore (11) having at least the six communicating opened parts (2) has at least two communicating opened parts (2) having a diameter of not less than 50 um, on the average.

3. The biomember according to claim 1 or 2, wherein a sum of a flat area of a globular pore (11) shown in any plain cross section and having a size larger than the mean pore diameter is not less than 25% and not more than 60% of the flat area of the total cross section.

4. The biomember according to claim 1 or 2, wherein a sum of a flat area of a globular pore (11) shown in any plain cross section and having a size larger than the mean pore diameter is not less than 35% and not more than 55% of the flat area of the total cross section.

5. The biomember according to any one of claims 1 to 4, wherein the sintered body is designed such that when in a dry state it is brought into contact with water or blood without pretreatment, water or blood infiltrates into a core part by a capillary phenomenon.

6. The biomember according to any one of claims 1 to 5, wherein a thickness of a circumference part of a communicating opened part (2) formed by causing a globular pore (11) to overlap with a globular pore (11) having a size larger than the mean pore diameter is set to be of about the thickness of a particle of calcium phosphate.

7. The biometer according to any of claims 1 to 6, wherein an active material (6) is attached on an inner surface of a globular pore (1).

8. The biomember according to claim 7, wherein an active material (6) is one chosen from a cell adhesion promoting material, cell proliferation promoting material, osteogenesis promoting material, bone absorption inhibiting material and vascularization promoting material, or combinations of at least two of cell adhesion promoting material, cell proliferation promoting material, osteogenesis promoting material, bone absorption inhibiting material and vascularization promoting material.

9. The biomember according to claim 7 or 8, wherein an osteogenic cell, automyelocyte, homogeneous myelocyte, fetal myelocyte, undifferentiated stem cell, osteogenic cell to which a gene of an active factor is introduced, automyelocyte to which a gene of an active factor is introduced, homogeneous myelocyte to which a gene of an active factor is introduced, fetal myelocyte to which a gene of an active factor is introduced, or undifferentiated stem cell to which a gene of an active factor is introduced, is introduced into a globular pore (1).

10. The biomember according to any one of claims 1 to 6, wherein drugs are stored in a globular pore (1) to be used as sustained release preparations.

## Patentansprüche

1. Bioelement zur Bildung eines Knochens in einem lebenden Körper, das aus einem porösen Körper eines Calciumphosphat-gesinterten Körpers, der eine Anzahl von im Wesentlichen globulären Poren (1) aufweist, und einem kompakten Teil besteht, wobei der kompakte Teil eine Porosität von nicht größer als 15 % hat, die Porosität des porösen Körpers nicht kleiner als 65 % und nicht größer als 85 % ist, und gleichzeitig der mittlere Porendurchmesser der globulären Poren nicht kleiner als 100 µm und nicht größer als 600 µm ist, wobei eine globuläre Pore (11), die eine Größe hat, die größer ist als der mittlere Porendurchmesser, zumindest vier kommunizierende geöffnete Bereiche (2) aufweist, die durchschnittlich einen Durchmesser von nicht kleiner als 5 µm haben, und gleichzeitig weist eine globuläre Pore (11), die zumindest vier kommunizierende geöffnete Bereiche (2) aufweist, zumindest einen kommunizierenden geöffneten Bereich (2) auf, der durchschnittlich einen Durchmesser von nicht kleiner als 50 µm hat, und gleichzeitig nimmt der gesamte Öffnungsbereich des kommunizierenden geöffneten Bereichs (2), den die globuläre Pore (11) aufweist, die eine Größe hat, die größer ist als der mittlere Porendurchmesser der globulären Poren, durchschnittlich nicht mehr als 40 % des Oberflächenbereichs der globulären Pore (11) ein, und im trockenen Zustand ist es möglich, das Gesamte durch Tropfen von Wasser und Blut zu benetzen,
wobei Mikropartikel von einer Größenordnung im Submikrometerbereich (d.h. nicht kleiner als 0,1 µm und bis zu 1 µm) als Rohmaterial verwendet werden, und in dem porösen Körper des Calciumphosphat-gesinterten Körpers der Partikeldurchmesser nicht größer ist als 5 Mikrometer,
wobei die globulären Poren (1) des porösen Körpers als Schaum gebildet werden, welcher durch Rühren einer Schlämme des Rohmaterials erhalten wird,
wobei der poröse Körper des Calciumphosphat-gesinterten Körpers Hydroxyapatit (8) ist,
wobei in eine globuläre Pore (1) Folgendes eingebracht ist, nämlich eine osteogenetische Zelle, eine Automyelocyte, eine homogene Myelocyte, eine fötale Myelocyte, eine undifferenzierte Stammzelle, eine osteogenetische Zelle, in die ein Gen eines aktiven Faktors eingebracht ist, eine Automyelocyte, in die ein Gen eines aktiven Faktors eingebracht ist, eine homogene Myelocyte, in die ein Gen eines aktiven Faktors eingebracht ist, eine fötale Myelocyte, in die ein Gen eines aktiven Faktors eingebracht ist, oder eine undifferenzierte Stammzelle, in die ein Gen eines aktiven Faktors eingebracht ist, wobei embryonale Zellen von den zuvor erwähnten Zellen ausgeschlossen sind.

2. Bioelement gemäß Anspruch 1, wobei die globuläre Pore (11), die eine Größe hat, die größer ist als der mittlere Porendurchmesser, zumindest sechs kommunizierende geöffnete Bereiche (2) aufweist, die durchschnittlich einen Durchmesser von nicht kleiner als 10 µm haben, und gleichzeitig eine globuläre Pore (11), die die zumindest sechs kommunizierenden geöffneten Bereiche (2) aufweist, zumindest zwei kommunizierende geöffnete Bereiche (2) aufweist, die durchschnittlich einen Durchmesser von nicht kleiner als 50 µm haben.

3. Bioelement gemäß Anspruch 1 oder 2, wobei eine Summe einer flachen Fläche einer globulären Pore (11), die sich in einer beliebigen Querschnittsebene darstellt, und eine Größe hat, die größer ist als der mittlere Porendurchmesser, nicht kleiner ist als 25 % und nicht größer ist als 60 % der flachen Fläche des gesamten Querschnitts.

4. Bioelement gemäß Anspruch 1 oder 2, wobei eine Summe einer flachen Fläche einer globulären Pore (11), die sich in einer beliebigen Querschnittsebene darstellt, und eine Größe hat, die größer ist als der mittlere Porendurchmesser, nicht kleiner ist als 35 % und nicht größer ist als 55 % der flachen Fläche des gesamten Querschnitts.

5. Bioelement gemäß einem der Ansprüche 1 bis 4, wobei der gesinterte Körper derart ausgestaltet ist, dass bei einem In-Kontakt-Bringen in trockenem Zustand mit Wasser oder Blut ohne eine Vorbehandlung das Wasser oder Blut einen Kernbereich durch einen Kapillarvorgang infiltriert.

6. Bioelement gemäß einem der Ansprüche 1 bis 5, wobei eine Dicke eines umlaufenden Teils eines kommunizierenden geöffneten Bereichs (2), gebildet durch Überlappenlassen einer globulären Pore (11) mit einer solchen globulären Pore (11), die eine Größe aufweist, die größer ist als der mittlere Porendurchmesser, derart festgelegt wird, dass diese in etwa der Dicke eines Partikels von Calciumphosphat entspricht.

7. Bioelement gemäß einem der Ansprüche 1 bis 6, wobei ein aktives Material (6) an einer inneren Oberfläche einer globulären Probe (1) angebracht ist.

8. Bioelement gemäß Anspruch 7, wobei ein aktives Material (6) ausgewählt ist aus einem Material, das die Zelladhäsion fördert, einem Material, das die Zellproliferation fördert, einem Material, das die Osteogenese fördert, einem Material, das die Knochenabsorption inhibiert, und einem Material, das die Vaskularisierung fördert, oder aus Kombinationen von zumindest zwei der folgenden Materialien, nämlich Material, das die Zelladhäsion fördert, Material, das die Zellproliferation fördert, Material, das die Osteogenese fördert, Material, das die Knochenabsorption inhibiert, und Material, das die Vaskularisierung fördert.

9. Bioelement gemäß Anspruch 7 oder 8, wobei in eine globuläre Pore (1) Folgendes eingebracht ist, nämlich eine osteogenetische Zelle, eine Automyelocyte, eine homogene Myelocyte, eine fötale Myelocyte, eine undifferenzierte Stammzelle, eine osteogenetische Zelle, in die ein Gen eines aktiven Faktors eingebracht ist, eine Automyelocyte, in die ein Gen eines aktiven Faktors eingebracht ist, eine homogene Myelocyte, in die ein Gen eines aktiven Faktors eingebracht ist, eine fötale Myelocyte, in die ein Gen eines aktiven Faktors eingebracht ist, oder eine undifferenzierte Stammzelle, in die ein Gen eines aktiven Faktors eingebracht ist.

10. Bioelement gemäß einem der Ansprüche 1 bis 6, wobei Arzneimittel in einer globulären Pore (1) gelagert sind, um als verzögert freigesetzte Zubereitungen verwendet zu werden.

## Revendications

1. Biomatériau destiné à former un os dans un organisme vivant, constitué d'un corps poreux fait d'un corps fritté de phosphates de calcium comprenant plusieurs pores substantiellement globulaires (1), et d'une partie compacte, dans lequel la partie compacte possède une porosité inférieure ou égale à 15 %, la porosité du corps poreux est supérieure ou égale à 65 % et est inférieur ou égale à 85 %, et simultanément, le diamètre moyen des pores globulaires est supérieur ou égal à 100 µm et est inférieur ou égal à 600 µm, un pore globulaire (11) possédant une taille plus importante que le diamètre moyen des pores possède au moins quatre parties ouvertes communicantes (2) possédant un diamètre qui est supérieur ou égal à 5 µm, en moyenne, et simultanément, un pore globulaire (11) qui possède au moins les quatre parties ouvertes communicantes (2) possède au moins une partie ouverte communicante (2) possédant un diamètre qui est supérieur ou égal à 50 µm, en moyenne, et simultanément, la zone d'ouverture totale de la partie ouverte communicante (2) qui appartient au pore globulaire (11) possédant une taille plus importante que celle dudit diamètre moyen des pores globulaires, n'occupe pas plus de 40 % de la surface dudit pore globulaire (11) en moyenne, et à l'état sec, il est possible de mouiller l'ensemble en y déposant des gouttes d'eau et de sang,
dans lequel des microparticules à l'échelle submicrométrique (c'est-à-dire allant de 0,1 µm à 1 µm) sont utilisées en tant que matériau brut, et dans le corps poreux fait d'un corps fritté de phosphates de calcium le diamètre des particules est inférieur ou égal à 5 micromètres,
dans lequel les pores globulaires (1) du corps poreux sont formés sous forme de mousse obtenue en agitant une pâte du matériau brut,
dans lequel le corps poreux fait d'un corps fritté de phosphates de calcium est de l'hydroxyapatite (8),
dans lequel une cellule ostéogénique, un auto myélocyte, un myélocyte homogène, un myélocyte foetal, une cellule souche indifférenciée, une cellule ostéogénique dans laquelle un gène d'un facteur actif est introduit, un auto myélocyte dans lequel un gène d'un facteur actif est introduit, un myélocyte homogène dans lequel un gène d'un facteur actif est introduit, un myélocyte foetal dans lequel un gène d'un facteur actif est introduit, ou une cellule souche indifférenciée laquelle un gène d'un facteur actif est introduit, est introduit dans un pore globulaire (1), moyennant quoi des cellules embryonnaires sont exclues des cellules mentionnées ci-dessus.

2. Biomatériau selon la revendication 1, dans lequel le pore globulaire (11) possédant une taille plus importante que le diamètre moyen des pores possède au moins six parties ouvertes communicantes (2) ayant un diamètre qui est supérieur ou égal à 10 µm, en moyenne, et simultanément, un pore globulaire (11) possédant au moins les six parties ouvertes communicantes (2) possède au moins deux parties ouvertes communicantes (2) ayant un diamètre qui est supérieur ou égal à 50 µm, en moyenne.

3. Biomatériau selon la revendication 1 ou 2, dans lequel la somme d'une zone plate d'un pore globulaire (11) présente dans toute coupe transversale lisse et possédant une taille plus importante que le diamètre moyen des pores ne représente pas moins de 25 % et pas plus de 60 % de la zone plate de la totalité de la coupe transversale.

4. Biomatériau selon la revendication 1 ou 2, dans lequel la somme d'une zone plate d'un pore globulaire (11) présente dans toute coupe transversale lisse et possédant une taille plus importante que le diamètre moyen des pores ne représente pas moins de 35 % et pas plus de 55 % de la zone plate de la totalité de la coupe transversale.

5. Biomatériau selon l'une quelconque des revendications 1 à 4, dans lequel le corps fritté est conçu de sorte que lorsqu'à l'état sec il est mis en contact avec de l'eau ou du sang sans prétraitement, l'eau ou le sang s'infiltre dans une partie centrale par l'intermédiaire d'un phénomène de capillarité.

6. Biomatériau selon l'une quelconque des revendications 1 à 5, dans lequel une épaisseur d'une partie circonférence d'une partie ouverte communicante (2) formée en faisant se chevaucher deux pores globulaires (11) possédant une taille plus importante que le diamètre moyen des pores est déterminée pour être environ de l'épaisseur d'une particule de phosphate de calcium.

7. Biomatériau selon l'une quelconque des revendications 1 à 6, dans lequel un matériau actif (6) est fixé sur une surface interne d'un pore globulaire (1).

8. Biomatériau selon la revendication 7, dans lequel un matériau actif (6) est un matériau choisi parmi un matériau promoteur de l'adhésion cellulaire, un matériau promoteur de la prolifération cellulaire, un matériau promoteur de l'ostéogenèse, un matériau inhibiteur de l'absorption osseuse et un matériau promoteur de la vascularisation, ou des combinaisons d'au moins deux matériaux choisis parmi un matériau promoteur de l'adhésion cellulaire, un matériau promoteur de la prolifération cellulaire, un matériau promoteur de l'ostéogenèse, un matériau inhibiteur de l'absorption osseuse, et un matériau promoteur de la vascularisation.

9. Biomatériau selon la revendication 7 ou 8, dans lequel une cellule ostéogénique, un auto myélocyte, un myélocyte homogène, un myélocyte foetal, une cellule souche indifférenciée, une cellule ostéogénique dans laquelle un gène d'un facteur actif est introduit, un auto myélocyte dans lequel un gène d'un facteur actif est introduit, un myélocyte homogène dans lequel un gène d'un facteur actif est introduit, un myélocyte foetal dans lequel un gène d'un facteur actif est introduit, ou une cellule souche indifférenciée dans laquelle un gène d'un facteur actif est introduit, est introduit dans un pore globulaire (1).

10. Biomatériau selon l'une quelconque des revendications 1 à 6, dans lequel des médicaments sont stockés dans un pore globulaire (1) dans le but d'être utilisés comme préparations à libération prolongée.
